# EUROPEAN PATENT SPECIFICATION

(11) **EP 0 469 021 B1**
(45) Date of publication and mention of the grant of the patent: **15.11.1995**
(21) Application number: 90906232.5
(22) Date of filing: 20.04.1990
(51) Int. Cl.: C12Q 1/02, C12Q 1/68, C12Q 1/66, C12N 15/69

(54) **DETERMINATION OF FACTORS AFFECTING GENE REGULATION AND/OR GENE REPLICATION**
BESTIMMUNG DER DIE GENREGELUNG UND/ODER GENREPLIKATION BETREFFENDEN FAKTOREN
DETERMINATION DES FACTEURS AFFECTANT LA REGULATION ET/OU LA REPLICATION DE GENES

(30) Priority: 20.04.1989 FI 891899
(43) Date of publication of application: 05.02.1992
(73) Proprietor: BIO-ORBIT OY, SF-20521 Turku (FI)
(72) Inventor: KARP, Matti, SF-20180 Turku (FI); KORPELA, Matti, SF-20810 Turku (FI)
(74) Representative: Fagerlin, Heléne
(86) International application number: PCT/FI90/00112
(87) International publication number: WO 90/12887

(56) References cited:
- EP-A- 0 121 386
- EP-A- 0 281 104
- SE-B- 0 424 090
- US-A- 4 806 471
- PROCEEDINGS OF THE NATL. ACADEMY OF SCIENCES USA, vol. 78, no. 8, August 1981, Washington, DC (US); H. DANBARA et al., pp. 4699-4703/
- NATURE, vol. 322, August 1986; M. PTASHNE (whole)/

## Description

Biotests are methods where one uses living cells or organisms as tools of detection different analytes. Many of those methods utilize bacterial or yeast cells. Procaryotic organisms and especially Escherichia coli bacterium are very well characterized. Yeast cells are eucaryotic ones growing anyhow as single cells. The cultivation of yeast is easier than the cultivation of higher eucaryotes. Yeast cells grow in simple cultivation mediums and they need not the addition of complicated growth factors. The knowledge of yeast is expanding rapidly and comprehensive maps of genes are known. Hundreds of specific mutations for both bacteria and yeast are known with which it is possible to study the activity of specific reactions and metabolic pathways. For instance with antibiotic sensitive bacterial mutants even trace amounts of antibiotics cause changes in the metabolism or in the membranes. Using these kind of mutants one is able to develope tests measuring residual antibiotics from biological material very sensitively. Mutants, whose cell membranes might be porous for different small molecular weight substances such as antibiotics. As well bacteria as yeast mutations where the repair mechanisms of their genetic material, DNA, are not working as well as with the wild-type strains are sensitive to genotoxic substances. Using different mutant strains one is able to measure for the presence of antibiotics and toxic or mutagenic agents.

It is rather simple to transfer new characteristics, proteins, into bacterial or yeast cells by genetic engineering techniques. These proteins, coded by virus, eucaryotic or procaryotic cell DNA, do not exist naturally in the target organism of gene transfer. This phenomenon expands the applicability of these organisms in biotests.

The relationship between carcinogenicity and mutagenicity is the basis for using mutagenic agents tests as prescreening tests for carcinogenic agents. The testing for carcinogenicity using test animals is extremely expensive and time-consuming. The testing of mutagenic agents as quick screening for also carcinogenicity has raised much hope and interest. One is thus able to decrease animal based carcinogenicity tests using rapid microbial tests. The basis is the universal genetic code in DNA whose structure and mode of action in each organism is similar.

Most often used test for mutagenic agents is AMES-test (Ames, B.N., McCann, J. and Yamasaki, E. (1975) Mutat. Res. 31, 347) utilizing Salmonella typhimurium bacterium as test organism. With this test one is able to detect the genotoxicity of most mycotoxins, aromatic amines and polycyclic hydrocarbons. However, Ames-test is not able to detect the genotoxicity of carcinogenic metal salts or chlorinated hydrocarbons. In the test the S. typhimurium strains used contain point mutations in the biosynthetic route of the amino acid histidine. As the bacteria are exposed to the action of mutagenic substance, a reversion phenomenon appears in the gene for histidine biosynthesis and the bacterium starts to produce histidine endogenously, thus giving the cell the ability to grow on minimal growth medium containing no added histidine. A pitfall in the test is poor sensitivity and slow performance. In this test all other genotoxic changes such as those acting on enzymes remain undetected. The test is also rather expensive for each particular compound to be tested.

A test for the detection of genotoxic substances based on bioluminescence has been developed (Ulizur, S., Weiser, I. and Yannai, S. (1980) Mut. Res., 74, 113-121). In this method dark mutants of Photobacterium leiognathi and P. fischeri are used. In the presence of genotoxic substances these strains start to emit light. The theoretical background of the method remains still somewhat obscure. It has been speculated that the effect of removal of a repressor or prevention of its formation combined to a change in chromosomal DNA of the bacterium might trigger the formation of light producing proteins. Different genotoxic substances act with different rate in this test due to the variety of different classes of substances. This test is faster than the Ames-test but is by no means easier to use. The bacteria used in the test should produce light during long and varying periods (30 min to 10 h) of time depending of the substance. The bacteria used are not capable of producing stable light emission, which makes the method somewhat problematic. Due to these facts the method is not easily automatized to be used for routine work when there are lots of specimens to be analyzed. Also being of marine origin the cultivation temperature of the bacteria in this method is rather low, 15°C - it is not known how well the effect of genotoxic substances correlate to the effects on man who's body temperature is at 37°C.

Antibiotics used as medicins against microbial invasion are detected from body fluids in order to study the dosage and penetration of the medicine. The effective therapeutic range of the antibiotic is often rather narrow and the risks due to overdosage might be big. It is also important to measure the presence/concentration of antibiotics from meat and cow milk due to symptoms in allergic people. The cow milk used in cheese production should not contain antibiotics due to the fact that cheese making bacteria are not to able use contaminated milk. The common methods for detecting antimicrobial medicines are microbiological methods performed on agar. A direct method is to measure the inhibition of the growth of sensitive bacterial strains. One can also measure some metabolic parameters such as acid production of a sensitive strain of bacteria using proper colour indicators.

Typical examples are cylinder, hole or disk methods to make agar diffusion tests. The difference between these tests is only restricted in the way the sample is applied on the agar and also in the way of usage of the bacteria in the test.

Since microbiological methods utilize bacteria or their spores it is the sensitivity of the test bacteria which is of utmost importance. So far one had to make compromises in the choice of a suitable test strain since great sensitivity against antimicrobial agents and other characteristics needed for the test strain have not been common features for the same strain of bacteria.

A major drawback when using microbes in antibiotic residues tests is the slow and unsensitive performance. In these methods one always controls in a way or other the growth of a tester strain one cannot imagine the test to be performed in an hour. This is due to the fact that growth of microbe is a slow phenomenon even at its fastest mode. Also in many cases one uses spores or freeze-dried microbes which makes the tests even more slow to perform.

Antibiotic detection methods based on bioluminescence measurement have been developed. Ulizur (1986, Methods Enzymol., 133, 275-284) describes three different ways to use bioluminescence for the detection of antimicrobial agents: a) lysis-test, b) induction test and c) bacteriophage test. In the first one the lux-genes isolated from Vibrio fischeri produce luciferase protein which in the presence of substrates produces light. The genes have been coupled into a plasmid, which has been transferred to Bacillus subtilis, which is sensitive to antibiotics affecting bacterial membranes such as penicillins and cephalosporins. In the test the B. subtilis containing the lux-genes is grown together with a sample. If there is an antibiotic present the synthesis of cell wall components is prevented and bacteria are lyzed, thus yielding a lower light emission level compared to blank.

In the induction test one uses dim mutants of P. phosphoreum bacteria, which do not produce light. This as well as other bioluminescence tests developed by Ulizur are based on exploitation of the chromosomal DNA of the target cell. With the induction test one is able to detect antibiotics affecting protein synthesis. When the bacteria are incubated together with compounds binding to DNA they start to produce light ie. protein synthesis is initiated. If there is any antibiotic present affecting the protein synthesis there is a decline in light emission. The amount of antibiotic present is quantitated when compared to blank without antibiotic. With this method one is not able to detect antibiotics affecting DNA synthesis. Also the actual performance of the method is questionable due to the fact that one does not know the basis of the method well. To perform the test it is essential to add minimal salts (such as Ca²⁺ - and Mg₂⁺ -ions) which are known to diminish or completely prevent the action of aminoglycosides (streptomycin, kanamycin, neomycin, erythromycin). Also the induction parameters are very strict and if samples contain other antibiotics (for instance nalidixic acid) or other substances triggering light production there might exist problems in the evaluation of the results. The difficulty in this test might be the great number of inducers. Also the amount of bacteria in the test has been claimed to be a critical parameter. If there exists a too high concentration of bacteria the test has to be aerated due to the absolute requirement of oxygen for the bioluminescence reaction in these bacteria. Problems occur when special measuring devices are used and the repetitity of the assay is affected due to these facts.

With the bacteriophage test one is able to detect antibiotics affecting DNA synthesis, transcription and translation. Wild-type, light-emitting P. phosphoreum bacteria are infected with lytic bacteriophages in this test. In the presence of antibiotic new infective phages cannot be synthesized due to the fact that DNA-, RNA- or protein synthesis is blocked. In the presence of antibiotic the light emission is unchanged compared to initial light level. However if there does not exist any antibiotic the phages rapidly multiply and inactivate the host bacteria thus making it incapable of producing light. Bacteriophage test is difficult to perform since it is necessary to add phages (sometimes with different titers) to the assay mixture and the timing of the addition of antibiotic has to be very careful. In this test same problems exist as with induction test in respect to the composition of assay mixture and the amount of bacteria used in the assay.

One is not able to detect a single antimicrobial chemical or groups of them with microbial methods for antibiotics used till now. Instead, these methods reveal all antibiotics to which the test microbe is sensitive. By changing measuring conditions or by adding enzymes degrading certain compounds one is able to block the effect of some antibiotics. There is a great demand for the detection of heavy metals, toxins or food additives simply and fast is great. At the moment determinations of those compounds have to be performed concentrated in central laboratories, since the devices for their determination are extremely expensive and need special trained personnel to use them. Quick, qualitative tests to be performed in the field could be remarkable filter for those samples which need more sophisticated instrumentation and research. Thus the pressure of central laboratories would be diminished and determination of problematic samples would be fastened.

A commercial "Microtox" test is able to detect toxic substances from environmental specimens. This test is based on the use of light emitting P. phosphoreum bacteria. Sample to be analyzed is incubated together with bacteria and the presence of toxic substance is evaluated from the lowered level of light produced by the bacteria when compared to controls. A severe drawback with this test is the need of high salinity (2 %) by the organism, which has been shown to decrease the biological effect of especially heavy metals. Also the measuring temperature of 15°C puts some obstacles. Also several tests utilizing whole animals or animal cell lines have been developed to measure toxic substances, but the pitfall in these methods is the complicated cultivation of cells, slow performance and need for skilled personnel.

Toxic and mutagenic substances should be able to be detected for example from different waters such as from waste-, consuming-, raw- and groundwater and from water for refreshment purposes Also water needed for industrial processes, food processes as well as raw water needed for pharmaceutical industry are of interest. Samples from ground sediments and air should be able to be detected for their toxicity and mutagenicity. The raw material used in food industry as well as quality control of food stuffs needs great attention. From certain waters one should be able to detect the organic material which could be used for instance to respiration and biosynthetic purposes of microbes contaminating the water. The organic material can be simple sugars, organic acids, peptides or proteins, compounds containing amino or phosphate groups linked to carbon chain etc. There is a need for rapid, non-expensive tests for these kind of compounds since the conventional methods take several days to completion in order to be able to evaluate the quality of water used for various purposes.

The invention described here is based on known and accepted principles on expression of genes and the factors affecting their regulation and on the use of these phenomenona in recombinant-DNA organisms such as bacteria and yeast.

Thus, a method of changing the copy number of a plasmid in a cell is known from the prior art. In Proc. Natl Acad. Sci., Vol. 78, No. 8, August 1981, H. Danbara et al.: "Regulation of DNA replication: "Target" determinant of the replication control elements of plasmid R6-5 lies within a control element gene", pages 4699 to 4703, high-copy-number mutant plasmids are used for quantitative analysis of the incompatibility expressed by these mutants for gaining information about the altered plasmid control system. This reference describes interaction of defined factors inside the cell, where said factors take part in the replication of the plasmid. The titration of replication inhibitor and developed in vivo determination described in the reference are used only for determination of certain natural inhibitor that already exists in the cell. The reference presents a new method for investigating a topic related to pure basic research, i.e. replication and factors related to its control.

Nature, Vol. 322, August 1986 Mark Ptashne: "Gene regulation by proteins acting nearby and at a distance", pages 697-701 relates to a review article describing the replication processes in detail.

EP-A-0 281 104 describes a plasmid vector comprising a trp promotor used as a vector for producing L-tryptophan.

EP-A-0 121 386 describes inducible plasmids intended for production of desired proteins.

However, none of the cited prior art documents discloses anything about a general analytical method for determining presence and/or quantity of an unspecific exogenous factor affecting the replication process.

The present invention provides for a method for determining the presence or amount of a factor in a sample to be tested, wherein the factor affects directly or indirectly the DNA, RNA and/or proteins of the cell or the synthesis mechanisms thereof, **characterized** in
a) incubating a sample to be tested with a population of transformed cells for a period sufficient to allow said factor, if present in the sample, to affect said cells, said cells being transformed with a recombinant-DNA plasmid, the replication of said plasmid being subject to a regulatable promoter, which can be induced by an exogenous stimulus independent of replication of the cells;
b) then applying said exogenous stimulus to said cells to induce replication of said plasmid, whereupon the copy number of the plasmid will begin to increase in the cell, if the factor has not affected the plasmid by inhibiting the replication; and
c) the change in the copy number of the recombinant-DNA plasmid in the cell is determined directly or indirectly, and compared with a reference test in which the factor was not present or in which it was present in a known amount, and thereby the presence or the amount of the factor is determined.

The present invention also provides for a method for determining the presence or amount of a factor in a sample to be tested, wherein the factor affects directly or indirectly the DNA, RNA and/or proteins of the cell or the synthesis mechanisms thereof, **characterized** in
a) incubatiang a sample to be tested with a population of transformed cells for a period sufficient to allow said factor, if present in the sample, to affect said cells, said cells being transformed with a high copy number recombinant DNA plasmid containing a DNA sequence encoding a marker protein or a part thereof essential to the biological activity of said protein, said sequence being coupled to a regulatable promoter such that expression of said marker protein is inducible by an exogenous stimulus;
b) then applying said exogenous stimulus to said population of cells to induce expression of said marker protein, whereby the amount of the marker protein in the cell starts to grow, if the factor has not affected the protein synthesis directly or indirectly;
c) determining the amount of the marker protein expressed by said population of cells, and comparing said amount with a reference test in which the factor was not present or in which it was present in a known amount, and thereby the presence or the amount of the factor is determined.

Gene technology has made it possible to use bacteria and yeast cells as hosts to produce even such kind of proteins that these organisms do not naturally produce. For these purposes several kinds of recombinant-DNA vectors have been prepared; most often then are extrachromosomal plasmids. Recombinant-DNA plasmids can contain several genes and they can proliferate independently. The technique utilizing rec-DNA is the most often used method to transfer foreign genes into new host cells. The gene in question can be joined to a plasmid vector by cutting the gene and plasmid-DNA with specific restriction endonucleases followed by the action of the enzyme DNA ligase which makes the covalent linkage between the gene and the vector. The transfer of hybrid plasmid-DNA into microbial cell can be performed by a transformation protocol whereby the cell wall of the target organism has been made permeable for the DNA. Several methods exist. When working with eucaryotic cell one uses rec-DNA plasmids which contain parts of DNA of procaryotic, virus and eucaryotic origin in suitable combinations. The transfer of DNA into eucaryotic cells is performed by for example Ca-precipitation or electroporation techniques.

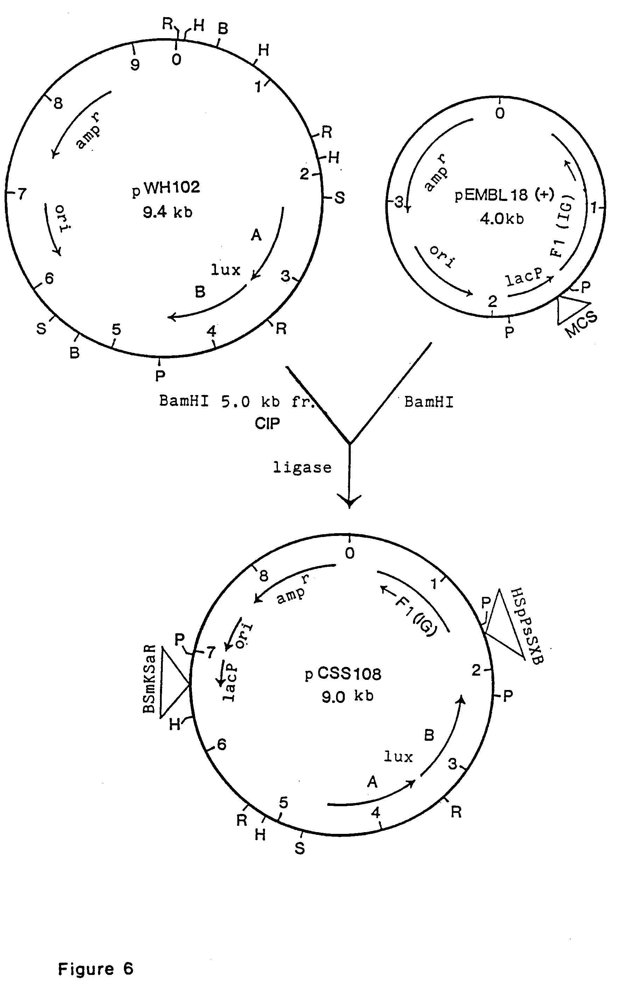

Many rec-DNA plasmids, where the production of foreign protein has been put under the control of strong promoter, have been developed during the last few years. In each case the goal has been to create as high production of foreign protein as possible in a new organism such as in E. coli. In these cases the production of foreign protein can yield as much as 25 % of total cellular protein (Caulcott & Rhodes, 1986, Trends in Biotech., June, 142-146). If such high amounts of protein are produced it is obvious, that this production is deletorious to the host cell and its metabolism. Due to the harmful effects several plasmids, where the production of foreign protein has been put under the control of a regulatable promoter, has been developed. The production of protein can here be turned on at the optimal growth phase of the microbe. In these cases the cultivation of the microbe is performed in unstressing conditions until growth has reached the cell density suitable for maximal production of the protein. The protein production is then switched on by adding a chemical to the medium which triggers the production. Also a change in physical parameters such as an increase or decrease in cultivation temperature might in certain cases cause the protein production. There is shown a plasmid pCSS108 (Korpela & Karp, Biotechnol. Lett., 10(6), 1988, 383-388) in Fig. 6, in which the production of bacterial luciferase can be switched on by the addition of chemical coupling agent isopropyl-β-D-thiogalactopyranoside (IPTG) in a cloned E. coli bacterium.

The plasmids commonly used contain one or more resistance factors, with which to select from large population of cells only those which contain the plasmid. The resistance factor helps the cell to survive in circumstances, which are poisonous to other cells. The selecting factor is added to the growth medium to prevent other cells growth except for the one containing the plasmid. The resistance determinant is a gene which codes for a protein which degrades or otherwise inactivates the poisonous factor (which can be for instance an antibiotic) present in the growth medium. Several genes encoding resistance factors are known, the one most often used is the gene coding for β-lactamase which is able to degrade penicillins, or β-lactams which are their derivatives. As the result the poisonous character of penicillin is lost and bacteria can grow. Other commonly used resistance genes are those coding for chloramphenicol acetyltransferase, kanamycin acetyltransferase and tetrahydrofolate reductase. Depending on the type of cells one uses also genes which carry the ability for the cell to grow in the presence of tetracyclin, erythromycin, spectinomycin, streptomycin, sulfonamides, neomycin, thiostrepton, viomycin and colisins. Some resistance factors which eliminate or change the heavy metal present in the medium are also known. Selection pressure in favor to cells containing a plasmid can also be achieved by transferring a gene encoding a function which complements a growth defect, which is lacking from the chromosome of the organism. These kind of genes are normally those which code for factors participating in amino acid biosynthesis pathways. In these cases a certain vital amino acid is in short in the growth medium and the cell can not grow unless the gene present in the plasmid produces the enzyme synthesizing the amino acid in question or its intermediate. Also other vital functions result in a beneficial situation for cells containing the plasmid compared to the cells without the plasmid. The plasmid can contain for instance a gene encoding a protein which participates in the formation of the cell wall components or heritable material.

The copy number of various plasmids inside the cell can vary from one to several hundreds, even for over thousand. The most often used plasmid pBR322 has a copy number of about 60 whereas a derivative of it pUC8 has a copy number of about 500. The reason for the high difference between two relative plasmids has been shown to be due to one base pair mutation in the origin of replication (ori) sequence of the plasmid (Chambers et al., 1988, GENE, 68, 139-149). One can artificially raise the copy number of a plasmid at suitable phase of growth by constructing a vector where ori has been put under the control of a strong and regulatable promoter. At present several plasmids are known whose copy number can be artificially shifted up during the growth of microbes. These plasmids are mainly used in industrial processes to produce foreign recombinant proteins in large quantities. Thus the use of these run-away replication vectors for purposes described above does not rule out the possibility of using them in this invention for measuring different agents affecting cell. As examples in this invention we describe different run-away plasmids with which the change in copy number is possible. Those studied and used most to produce foreign proteins are plasmids belonging to series pOU where the origin of replication region has been put under the control of strong and regulatable p_{R} promoter of phage lambda (Larsen et al., 1984, GENE, 28, 45-54). The p_{R} promoter of phage lambda is regulated by the repressor protein cI857, which is destroyed by heat treatment to 42°C. The production of this protein can be done from a lysogenic phage, from a phage which is conjugated to chromosome of the host cell, from a plasmid where the coding sequence has been introduced or from an other plasmid which belongs to a different incompatibility group. In this context by different incompatibility groups of plasmids are meant plasmids which are able to replicate independently without the presence of another plasmid in the same cell. When the repressor protein has been destroyed, p_{R} promoter is turned on and without control it starts to produce proteins called copB and repA (originating from low-copy number plasmid R1) as well as transcription products of these and the copA gene. These factors and especially the strong overproduction of repA protein result in enhanced or even uncontrolled production of the plasmid-DNA in E. coli bacterium.

Yeasts as well as bacteria are single cell organisms but yeasts differ from bacteria by being representatives of eucaryotic cells. Compared to higher eucaryotic cells yeasts are far better characterized from the genetical point of view. The genetic maps of Saccharomyces cerevisiae and Schizosaccharomyces bombei are already known in great detail (Petes, 1980, Ann. Rev. Biochem., 49, 845-876). Also powerful methods to transfer genes into yeast are known. Indeed, yeasts are commonly used hosts of rec-DNA.

There exists four types of rec-DNA vectors used with yeasts: integration plasmids (YIₚ), episomal vectors (YEₚ), replicating vectors (YRₚ) and artificial chromosomes. The integrating vectors of yeast can contain DNA originating from bacteria and part(s) of yeast genes. This type of plasmid binds exactly on certain point(s) in the yeast chromosome. The replicating yeast plasmids contain DNA from bacteria, part of yeast DNA and a specific area from yeast chromosome, which is responsible for the replication of the plasmid.

This area permits the plasmid to replicate as extrachromosomal DNA molecule in the yeast cell. The episomal plasmids contain DNA from bacteria, a yeast gene and a part or the whole 2 micron plasmid of yeast (Hollenberg, 1982, Current Topics in Microbiology and Immunology, 96, 119-144). Artificial chromosomes are linear DNA vectors which are not well suited for expression of heterologous proteins. A plasmid structure for yeast has been described whose copy-number can be regulated. The centromers of yeast are needed during the partition of chromosome in mitose and meiose phases. Centromeric DNA (CEN3) has been extracted and transferred under the control of alcohol dehydrogenase promoter (ADH2) which is repressed by glucose. The action of this kind of a plasmid CEN3 can be controlled by the carbon source used to cultivate yeast. When glucose is used as carbon source the ADH2 promoter is repressed and then CEN3 works normally by balancing the plasmid structure (YRₚ) during mitoses. If the carbon source is changed in the growth medium the plasmid starts to replicate in the cell and the copy number can rise up to one hundred per one yeast cell (Chlebowicz-Sledziewska, E. & Sledziewska, A., 1985, GENE, 39, 25-31).

The expression vectors used in yeasts contain normally the following strong regulatable promoters: alcohol dehydrogenase isoenzyme I (ADHI) gene promoter, phosphoglycerol kinase (PGK) promoter, repressible acid phosphatase (PHO5) promoter and the promoter for α-factor. ADHI is a cytoplasmic enzyme of yeast, which produces ethanol from acetaldehyde and needs NADH as a cofactor. When yeast cells are cultivated in the presence of glucose there is at least 1% ADHI protein from the total amount of proteins in yeast. The promoter of PGK can be controlled by the carbon source (for example glucose) used, which activates the expression of the protein controlled by the promoter. The expression of PHO5 can be prevented by the addition of inorganic phosphate and again activated by eliminating inorganic phosphate from growth medium. The control of PHO5 happens through special regulation apparatus, which is formed from PHO2, PHO4, PHO80 and PHO85 gene products (Bosfiana, K.A., 1980, Proc. Natl. Acad. Sci. USA, 77, 6541-6545). Also some mutants (PHO4 and PHO80) are known, which can be activated by a simple change in temperature. These mutant yeast cells grow at 35°C and do not produce acid phosphatase enzyme even if inorganic phosphate does not exist in the medium. If the cultivation temperature is shifted down acidic phosphatase is produced efficiently wheather there is phosphate or not in the medium. This system to control the protein production by a change in the cultivation temperature has been used to produce for instance interferons (Kramer et al., 1984, Proc. Natl. Acad. Sci USA, 81, 367-370).

In order to produce foreign proteins the use of higher eucaryotes as host cells for rec-DNA vectors is rapidly expanding. The wish is to produce proteins of eucaryotic origin in high quantities. In an optimal expression system it would be possible to produce proteins in several different types of cell lines. A fully regulatable expression system for protein production would be an ideal solution. Most often used regulatable promoters work only in certain host cell systems. Often the regulation of these promoters is poor and the expression vectors are based on DNA of tumor producing viruses, thus there exists also certain risks in their uses.

In higher eucaryotes the gene expression can be regulated with the help of following means: simian virus (SV40) T-antigen, metallothionein genes, heat-shock genes, glucocorticoid hormones, DNA methylation or with anti-sense RNA. The antigen produced by SV40 controls its own transcription. T-antigen is produced in high amounts immediately after the virus has infected the target cell and later the T-antigen binds to its own promoter and prevents the transcription. If SV40-vectors are used for cloning the regulation of the T-antigen can be prevented by using a suitable temperature sensitive T-antigen mutant. In these cases T-antigen mutants produce T-antigen normally at high temperatures but the production is prevented at room temperature (Rio et al., 1985, Science, 227, 23-28). Metallothioneins are proteins which bind heavy metals. Many eucaryotic cells produce these proteins in the presence of heavy metals. It has been estimated that there is an increase over fifty fold in the production of metallothioneins when cadmium was added to the growth medium to the concentration of 4x10-6 molar (Hamer, D.H. & Walling, M.J., 1982, J. Mol. Appl. Genet., 1, 273-288). The protein production induced by cadmium can be further increased by using low Cd²⁺-content growth media.

Many promoters of heat-shock genes have been shown to be applicable and well regulated in several different cell lines. The regulation of these promoters is performed simply by shifting the growth temperature. The genes are activated to produce proteins at high temperatures, on the other hand at low temperatures the proteins are produced in low amounts or not at all. The best studied case is the heat-shock system of colon fruit-fly, Drosophila melanogaster, in which the rise of temperature from 25°C to 37°C causes the ceasing of normal protein production whereas the heat-shock proteins start to emerge. The major and best known heat-shock protein is called hsp70. The regulation mechanisms of the expression of the proteins are not well known. By using heat-shock promoters (hsp70) it has been possible to increase the production of hGH (human growth hormone) up to 1200-fold compared to unactivated cells (Dreano et al., 1986, GENE, 49, 1-8).

In the invention described here advantage is taken of procaryotic and eucaryotic organisms, which have been carefully selected and which contain applicable rec-DNA vector constructions. By turning on the synthesis of DNA, RNA or proteins under strict control one is able to measure or detect either directly or indirectly all those factors which affect on the synthesis machineries described above. As the basis of measurement one can use the protein product encoded by the rec-DNA vector, the marker protein or its activity or the overall metabolic activity. By activating the replication of rec-DNA vector in a controlled fashion one is able to measure the amount of DNA formed also directly using radioactive labels or with flow-cytometric techniques.

One is able to prepare suitable rec-DNA vectors for the measurement of different classes of chemicals depending on what is the target of the chemical. It is possible to quantitate for instance the compunds inhibiting DNA synthesis (nucleotides) and DNA replication as well as those compounds binding to DNA like several cancer drugs by the aid of E. coli bacteria containing runaway-replication type plasmids. The replication of DNA in these kind of plasmids is controlled for instance by an inducible promoter p_{RE} of phage lambda. Thus the DNA synthesis and the replication of plasmid can be triggered at a predetermined point of time and the analytes to be measured can be linked directly to this regulatable and strong biosynthesis of DNA which is not dependable on cell division. If the synthesis or replication of DNA is inhibited the result is seen as the copy number of plasmid is being the same or even decreased compared to the initial stage. In the uninhibited control cells the copy number of plasmid per cell is increasing rapidly. The change in copy number can be measured either directly by measuring the amount of DNA or indirectly by measuring the amount of gene products or the activity coded by the plasmid DNA. It is possible to determine agents that have very different mode of action on the cell with the aid of this kind of a plasmid. This is due to the fact that one can also engineer a gene encoding a marker protein under the control of a strong and regulatable promoter the expression of which is measured in the test. Thus everything affecting DNA, RNA, proteins, their biosynthesis in a way or another, can be measured. If one wants to develop a broad range test which covers agents affecting cell wall, nucleic acids, proteins and metabolism an ideal means of detecting these agents is based on this kind of a runaway-replication plasmid. In these cases cells are allowed to replicate after which the promoter regulating the replication is activated and simultaneously or after a certain period also the promoter regulating the gene encoding the marker protein is activated. A vector with similar characteristics can be developed for eucaryotic cells.

Another kind of approach is to use plasmids whose replication is tied to host cell division. Rec-DNA multicopy plasmids in which the gene encoding the marker protein is under the control of a strong and regulatable promoter can be used to detect agents that affect cell membranes, proteins and metabolism. Agents affecting DNA or cell membranes can be detected with the system if actively dividing cells are used. The multicopy plasmid is synthesized to daughter cells and the system is sensitive to agents affecting DNA. Actively dividing cells are also sensitive to agents affecting the cell membranes. If the strong promoter regulating the expression of the marker protein is activated the system will then also be sensitive to agents affecting the mRNA and protein synthesis.

As a special application, when genes encoding luciferase are used, there is also a possibility to determine agents that affect energy metabolism. This is due to the fact that the reactions catalyzed by luciferases use energy-rich substances of cells. Agents that can affect the energetic state of the cell on all biosynthetic levels (replication, transcription and translation) or in metabolism, can be determined with the aid of bioluminescence ie. formation of light emission by the cells. A special case is bacterial luciferase, which uses central products of metabolism, NAD(P) and FMNH₂. Another special case is fire-fly and click beetle luciferases, which use central metabolite, ATP, for light production.

The nature of the invention described here makes it possible to use very different kind of measuring modes for example spectrophotometric, fluorometric, luminometric and visual methods. Spectrophotometric methods can be an alternative when there is a gene cloned into plasmid whose product can be measured by monitoring the change in colour such as β-galactosidase, alkaline phosphatase, amylases, peroxydases, glucuronidases or oxidoreductases. Fluorometric methods utilize fluorescent substrates developed for various enzymes, thus yielding somewhat greater sensitivity compared to spectrophotometric techniques. The luminometric method is performed with the aid of genes encoding either bacterial or beetle luciferases. There exist several luminescent bacterial species such as V. harveyi, V. fischeri, P. leiognathi, P. phosphoreum, Xenorhabdus luminescens etc. Luminescent beetles are for example Luciola mingrelica, Photinus pyralis, Pyrophorus plagiothalamus etc. There exist also several eucaryotic species in the sea which luminesce, such as marine ostracod Vargula hilgendorfii, jellyfish Aequorea victoria, batrachoidid fish Porichtys notatus, pempherid fish Parapriacanthus ransonneti etc., which could be useful in the future for various applications. Here an advantage over spectrophotometric and fluorometric measurement is the extremely sensitive detection of light emission. An important benefit in luminescent methods is the possibility to calibrate internally the measurements by using inside the same cell other genes which encode luciferase emitting a different colour which could be measured with special two wavelenght-detecting apparatus. The other gene can be cloned in the same rec-DNA vector, in an other vector belonging to a different incombatibility group, inserted in the host chromosome, it can be carried in a phage etc. An example is the click beetle luciferases, which emit four different colours the wavelenghts ranging from 547 nm to 593 nm (Wood et al., 1989, Science, 244, 700-702). The other gene resulting in different wavelenght can be put under inducible production system (indicator "gene") or it can be expressed constitutively (internal standard) to compensate possible secondary effects arising from heterologous samples. The use of simple colour indicator is useful in cases where there is no need for high sensitivity but where the simplicity and fast performance are more important. If the changes in cell metabolism are to be detected one can use for example tetrazolium salts which form a low-solubility formazan colour when reduced. In these cases genes encoding dehydrogenases or oxidoreductases act as mediators of reducing quantities to yield the intense colour of formazan. Also immunological methods (antibodies) coupled to sensitive measuring systems (RIA, FIA) are possible. Use of radioactive labels and flow cytometry in detecting the end point of the test are possible.

### The method based on the change in copy number of rec-DNA vector:

The cell builds its heritable material, DNA, from deoxyribonucleotides. There can exist also extrachromosomal or episomal DNA as plasmids in the cell. The replication of the plasmids is not directly dependable on the cell proliferation. Each plasmid has its own origin of replication by which to replicate and divide into daughter cells in the course of cell division. However, the plasmid uses the host cell's DNA replication machinery for its own replication.

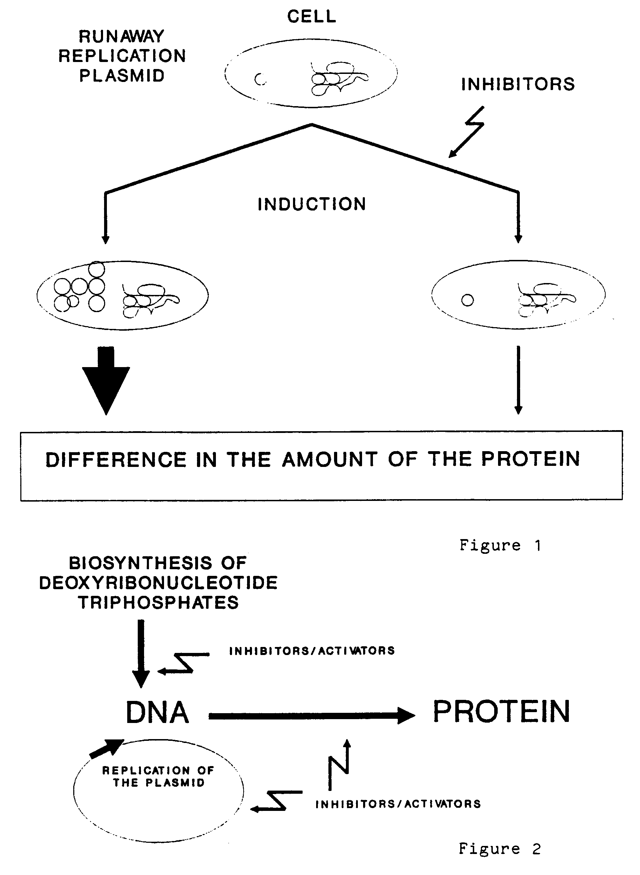

In Figure 1 is shown a schematic representation of a method based on the change in plasmid copy number in which the cell containing a special plasmid (for example runaway-replication plasmid pCSS123) can be made replicating at a predetermined point of time in a controlled fashion. In the beginning the cell contains only few copies of the plasmid. The agent to be examined is allowed to affect the cell for a suitable period of time after which the replication of the plasmid is commenced. The plasmid will then replicate as much as possible in the presence of the agent. The replication of the plasmid can be triggered by adding a coupling chemical or by physical means like shifting the temperature high enough for replication to commence. Simultaneously or after triggering the replication the expression of the marker protein can be turned on from the same special plasmid. In this case the degree of the plasmid replication can be directly quantitated by measuring the amount of the marker protein or its activity which is dependent on the copy number of the plasmid inside the cell. This has been described in Figure 1 as an amount of enzyme activity produced by the plasmid encoded gene. This makes it possible to study and measure factors affecting the synthesis of RNA, transcription, translation, cell walls, specific metabolic pathways and enzyme activities as well.

There is shown in Figure 2 a schematic representation on the possibilities to have an effect in the cell with different agents and how they can be coupled to a change in plasmid copy number. The biosynthesis of DNA, RNA and protein are multistep procedures and they need the cooperation of several factors. Each step has both natural and artificial agents which affect the systems either by activating or inhibiting them. For instance nalidixic acid has an effect on the replication of DNA by inhibiting the action of DNA polymerase.

A remarkable thing is that the starting point is few regulatable DNA molecules which can be made to replicate without cell proliferation. This makes it possible to use unproliferating cells for the testing of effectors so that the time used for the assay is not limited by the slow growth and proliferation of cells. The inventiveness of this method is based on controlled multiplication of the plasmid DNA and therefore on a possibility to investigate very large groups of compounds.

In the invention advantage is taken of regulatable promoters and machineries controlled by these promoters such as the increase of a copy number of a plasmid and/or production of a marker protein by the cell at a predetermined phase of growth. This context promoters are meant in areas of DNA where the enzyme RNA polymerase can bind, and where a special regulator protein or other molecule can interact as well. Promoters are quantities in the DNA which control the expression of a gene beside or nearby it. Inducible E. coli promoters are for example lac, trp, hybrid promoter tac and p_{L} and p_{R} promoters of phage lambda. These promoters differ in respect from each other for instance in strenght and in mode of induction. lac and trp promoters can be induced with chemicals whereas induction of p_{L} promoter can be started by a simple heat treatment.

### Determination of toxic substances using a method where protein biosynthesis is controlled by a regulatable promoter in rec-DNA plasmid:

Another material containing information in cells than DNA is RNA, especially messenger RNA (mRNA). Messenger RNA is synthesized from ribonucleotide triphosphates, which are stored in the cell. Messenger RNA is synthesized according to each gene in the DNA in a special transcription machinery which contains molecules responsible for this action. On the other hand proteins are made according to mRNA-molecule templates using universally accepted principles. RNA synthesis can be switched on very fast as well as the synthesis of a protein coded by the corresponding RNA. In the invention there are used special rec-DNA plasmids which have been prepared so that they can be activated to produce suitably selected proteins in high quantities. In this case a special plasmid has been constructed so that the copy number of the plasmid cannot be selected but it is constant for the plasmid used in certain host cell. Plasmids to be used should be of high copy number and consequently the production of protein would be high. As cells containing these kind of plasmids are treated with agents such as antibiotics affecting mRNA or protein synthesis before induction one could estimate from altered protein production the amount of antibiotic, mode of action or overall presence in the system. The idea of the invention is here applied to such cases where microbes are exposed to agents which affect biosynthesis routes which are induced specifically and strictly controlled in the presence of these agents. As rec-DNA plasmids are used as described in the invention one is able to get the selected protein production dependent on the agents used.

Remarkable is that one is able to determine agents affecting DNA synthesis and cell membranes. This is possible due to the fact that when a microbe is proliferating it is forced to synthesize these high-copy number plasmids and hence these special plasmids are susceptible to these agents. Actively proliferating cells are especially susceptible to agents acting against cell membranes and their biosynthesis.

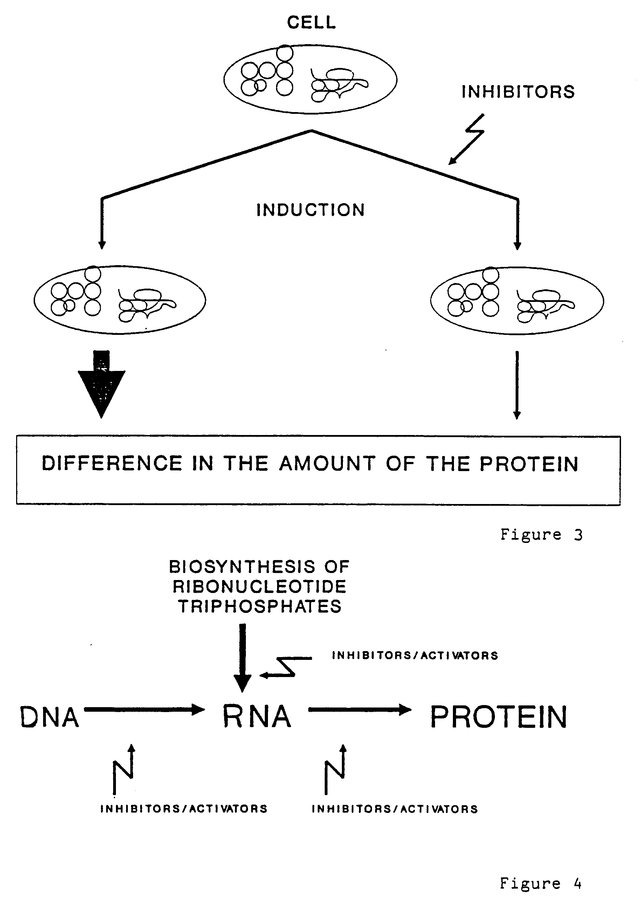

There is shown in Figure 3 the practical performance of the invention described above in case where the special plasmid is existing in many copies in the microbe. The microbe is exposed to an agent inhibiting biosynthesis and after a period of time the special plasmids are activated to produce protein, which in this case is an enzyme. The thickness of the arrows shown in the Figure are corresponding to the efficiency of mRNA and protein synthesis and thus also to the efficiency of the agents. In the case where the inhibiting agent has been present the production of the enzyme has remained low. When compared to the case, where affecting agent was not present or there were known amounts of it one is able to perform the measurement either quantitatively or qualitatively.

It is shown in Figure 4 in simpler format those biosynthetic routes which can be affected with the rec-DNA plasmid described above if non-proliferating cells are not used. If one uses actively proliferating cells in the measurement it is also possible to study agents affecting DNA as it is shown in Figure 2. The difference to Figure 1 is the initial amount of plasmid copies in the cell and the possibility to actificially rise the copy number in non-proliferating cells.

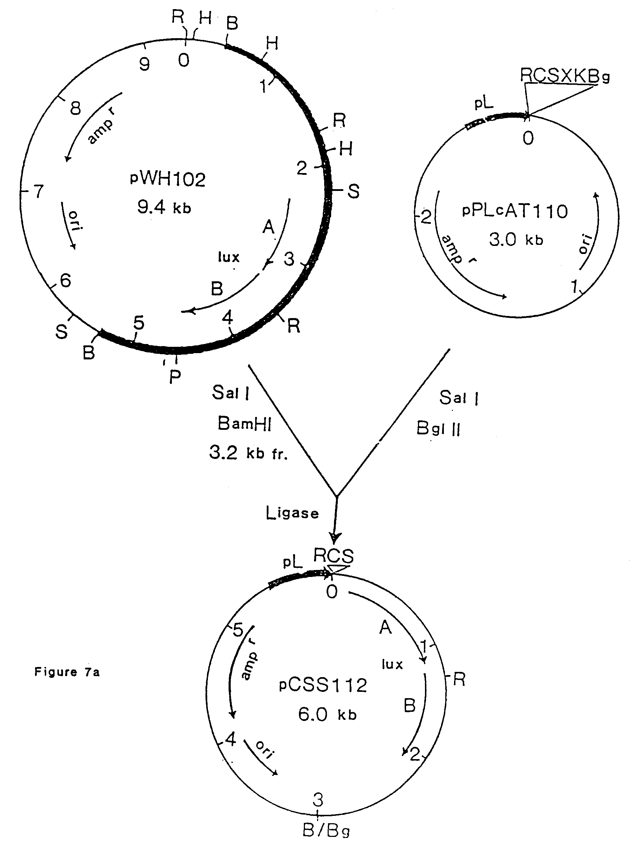

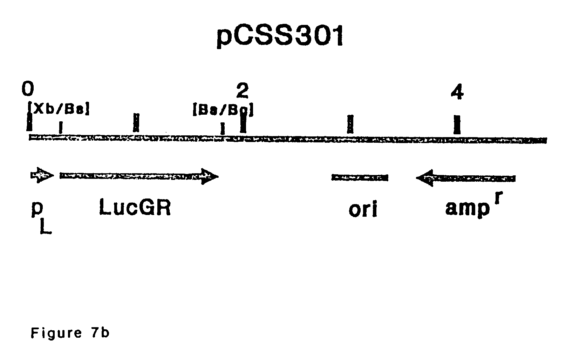

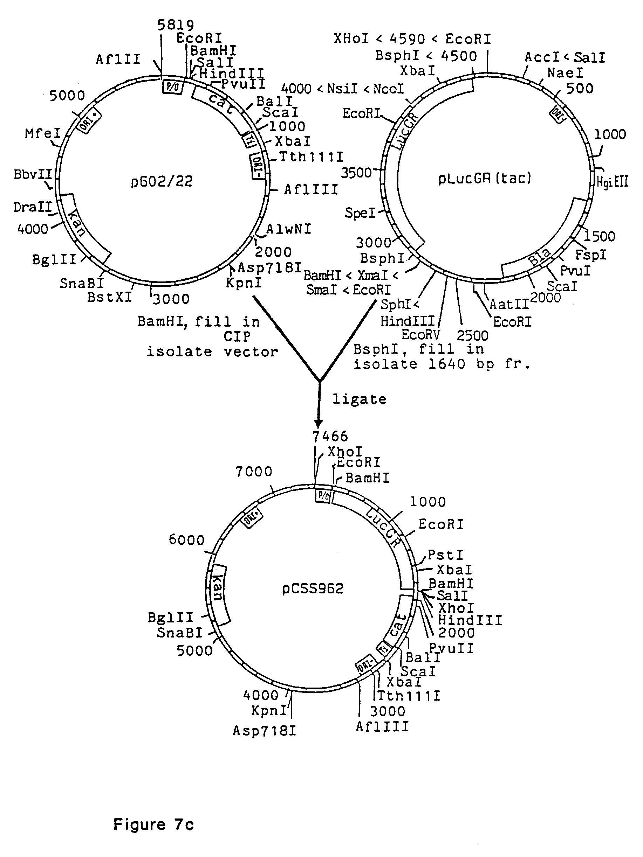

There exist promoters, which can be switched on by suitable treatments. The strenghts of promoters vary a lot and every promoter described in this invention is rather strong. However, one can say that p_{L} promoter is stronger than lac and trp promoters. The p_{L} promoter of phage lambda is also much faster ie. the effect of induction is clearly seen much earlier compared to the other two. The rate can be partly explained by the slow incorporation of inducing molecule through cell membranes in the case of lac and trp promoters and further to the effector site inside the cell (for example it can be mentioned the inducer of lac promoter, IPTG). Also the copy number of the plasmid used partly explains the relative differences of the induction. The copy number of a plasmid pCSS112 (see Figure 7) in E. coli is about 60, whereas plasmid pCSS108 (see Figure 6) has a copy number of around 600. The production of bacterial luciferase by the plasmids is controlled in pCSS112 by the p_{L} promoter of phage lambda and in pCSS108 by the lac promoter of E. coli lactose operon. Both promoters are controlled by certain repressor proteins, which are produced in limited amounts. As the copy number of plasmid in the former case is ten times lower than in the latter case the production of luciferase protein is better shut down ie. repressed. In the latter case the lac promoter leaks due to the relatively low amount of repressor protein and thus the grown level of the protein to be determined or its activity is already on high level. The effect of toxic substances can be shown more effectively when a strong and fast-induced promoter is used to regulate a certain gene or action in a rec-DNA vector.

### The bacterial strains, plasmids and their construction, methods used in the invention:

As cloning hosts and in toxicity measurements E. coli JM 103 (lac-pro, thi, strA, supE, endA, sbsB15, hsdR4 (F'traD34, proAB, lacI^{q}ZλM15) (Messing et al., Nucl. Acids Res., 9, 1981, 309-321), MC1061 (cI⁺, araD139, λ(ara-leu)7696, lacX74, galU⁻, galK⁻, hsr⁻, hsm⁺, strA) (Casadaban & Cohen, J. Mol. Biol., 138, 1980, 179-207), BW322 (CGSC, rfa-210::Tn10, thi-1, relA1, spoT1, pyrE) and K-12 (M72 Sm^{R}-lacZamλbio-uvrB, trpEA2 (Nam7Nam53cI857 HI) (Remaut et al., 1981, GENE, 15, 81-93) and Bacillus subtilis 1A40 (Bacillus Genetic Stock Center, lys-3, metB10, trpC2) were used. Cells were grown on appropriate minimal agar plates and were kept maximally one month at +4°C after which new plates were stroken. The strains were kept also in 15% glycerol at -70°C, wherefrom growth was started through minimal plates. Cells for plasmid extraction were first cultivated in 5 ml of 2xTY medium (16 g Bacto tryptone, 8 g Yeast extract, 8 g NaCl, H₂O ad 1 l, pH 7.4, with appropriate antibiotic) 10 h at 30°C in a shaker after which the cultivation was transferred to a bigger volume for 10 h same medium.

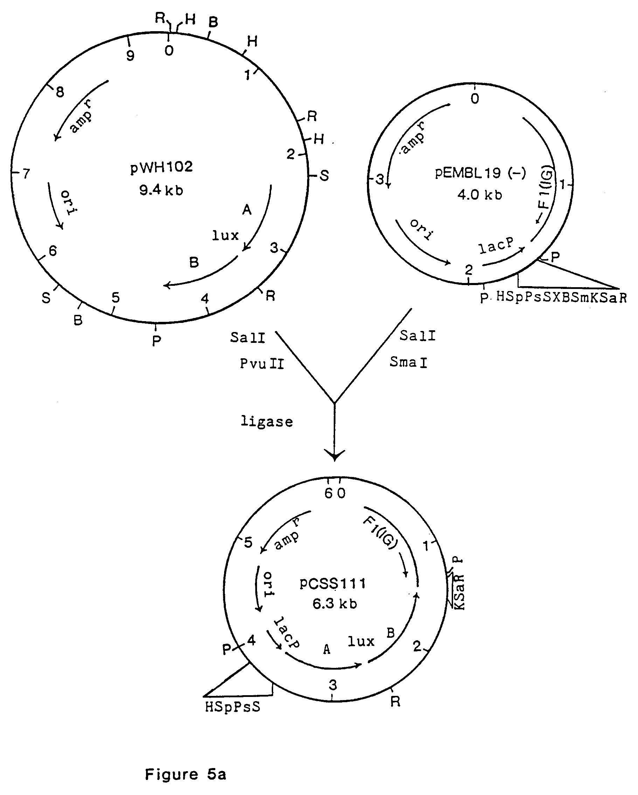

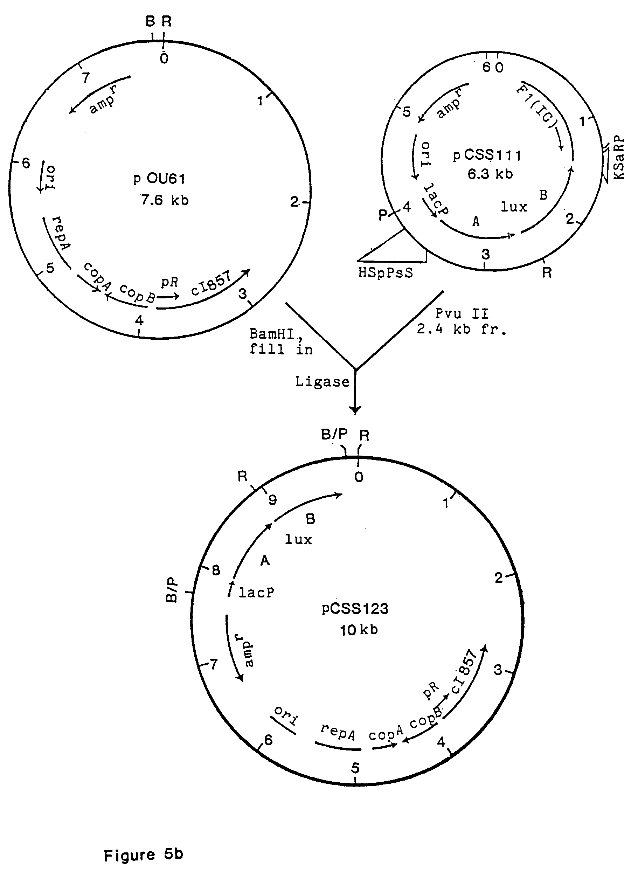

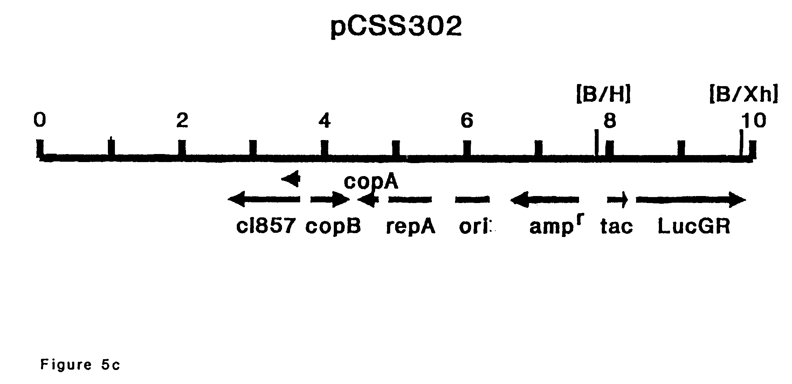

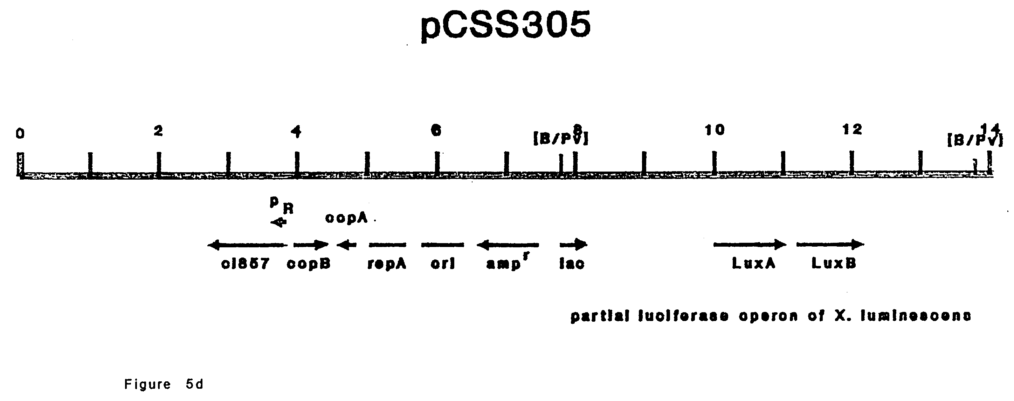

There is shown in Figures 5a and 5b the construction of a rec-DNA plasmid pCSS123 (deposited with a DSM number 5119), in Figure 5c the construct of a rec-DNA plasmid pCSS302 and in Figure 5d the construct of a rec-DNA plasmid pCSS305. Plasmid pWH102 (Gupta et al., 1985, Arch. Microbiol., 143, 325-329) was cut with the restriction enzymes SalI and PvuII and it was driven in agarose gel electrophoresis. A DNA band of 2300 base pairs (bp) was cut under UV light and the low-gelling temperature agarose was melted at 65°C and ligated with ligase enzyme to a plasmid pEMBL19(-) (Dente et al., 1983, Nucleic Acids Res., 11, 1645-1655) which had been cut with SalI and SmaI. The plasmid obtained was transformed into E. coli JM103 cells using the method described later. A plasmid extraction in mini-scale was performed according to Maniatis et al. (1982, Molecular Cloning: A Laboratory Manual, Cold Spring Harbor Laboratory, Cold Spring Harbor) and the correct constructions were verified with suitable restriction enzyme analysis. Plasmid extraction in large scale was performed according to the same manual. The plasmid shown in Figure 5a (pCSS111) was cut with the restriction endonuclease PvuII and a DNA-piece of 2400 bp was separated as described earlier. This piece containing bacterial luciferase genes from V. harveyi under lac promoter control was ligated to a plasmid pOU61 (Larsen et al., 1984, GENE, 28, 45-54) cut with the restriction enzyme BamHI which was first filled in with DNA-polymerase Klenow enzyme in a proper buffer system. Ligation mixture was transformed to E. coli JM101 strain as described later and correct plasmid containing colonies were picked by their ability to produce light after visual checking of the plates in dark room. This was performed by adding 5 µl of 10 % decanal on the lid of cultivation plate, which revealed the light producing colonies after few minutes after the aldehyde had penetrated the cells. The plasmid obtained is shown in Figure 5b and was named as pCSS123. An analogous runaway-replication plasmid pCSS302 was constructed as follows: Plasmid pLucGR(tac) (Wood et al., 1989, Science, 244, 700-702) was cut with the restriction endonucleases XhoI and HindIII and filled in with Klenow enzyme. After separation of the fragments on agarose gel a 1800 bp fragment containing the gene encoding green luciferase of click beetle under the control of tac promoter was ligated to plasmid pOU61 which was cut with BamHI and filled in as described above. Ligation mixture was transformed in E. coli JM103 and correct transformants were verified from plasmid minipreparations and the resulting plasmid pCSS302 is shown in Figure 5c. A second analogous runaway-replication plasmid pCSS305 was constructed as follows: Plasmid pCGLS11 (K. Nealson, personal commucication and in press) was digested with the restriction enzyme PvuII and a 7 kb fragment containing the genes encoding luciferases α and βsubunits of X. luminescens under the control of lac promoter of E. coli were ligated to plasmid pOU61 which was cut with BamHI and filled in as described above. Ligation mixture was transformed in E. coli JM103 and correct transformants were verified from plasmid minipreparations.

The symbols and abbreviations used: amp^{r} = gene encoding β-lactamase, ori = the origin of replication of the plasmid, luxA and B = genes encoding the subunits of luciferase, lacP = the promoter of the lactose operon of E. coli, F1(IG) the intergenic region of phage F1, MCS = multible cloning site of pUC18 (Yanisch-Perron et al., 1985, GENE, 33, 103-109), kb = thousand base pairs, repA, copA and cobB = genes encoding proteins responsible for plasmid copy number and partitioning of plasmid in daughter cells, cI857 = the temperature sensitive repressor of phage lambda. The abbreviations of restriction endonucleases used: R = EcoRI, H = HindIII, B = BamHI, S = SalI, P = PvuII, Sa = SacI, K = KpnI, Sm = SmaI, X = XbaI, Ps = PstI, Sp = SphI. B/P = the ligation point BamHI, filled in, PvuII. B/H = ligation point BamHI, filled in, HindIII. B/Xh = ligation point BamHI, filled in, XhoI.

There is shown in Figure 6 a plasmid pCSS108 (Korpela & Karp, Biotechnol. Lett., 10, 1988, 383-388), which is used for production of bacterial luciferase by adding a chemical called IPTG, which triggers protein production by binding to lac repressor protein. The genes encoding luciferase from Vibrio harveyi were transferred from the plasmid pWH102 (Gupta et al., 1985, Arch. Microbiol., 143, 325-329) by cutting the plasmid with the restriction enzyme BamHI. The two pieces obtained were treated with enzyme alkaline phosphatase (CIP) to remove the terminal phosphate groups so that the pieces can not ligate to themselves. A piece of 5000 bp was separated on agarose gel as described previously. This fragment was ligated to a plasmid pEMBL18(+) which had been previously cut with the same enzyme by using T4-DNA ligase. After transformation to E. coli JM103 and overnight incubation of the transformants the cultivation plates were screened for light producing colonies as described previously in dark room. Symbols used are as in Figures 5.

The construction of plasmid pCSS112 (deposited as a DSM number 5120) is shown in Figure 7a. The plasmid contains luciferase genes from V. harveyi and they are under the control of the p_{L} promoter of phage lambda. The promoter is regulated by the repressor protein cI857 of phage lambda, which can be destroyed by short heat treatment in suitable bacterial host such as E. coli K-12 HI trp. Plasmid pWH102 was cut with restriction enzymes SalI and BamHI. Plasmid pPLcAT110 (Stanssens et al, 1985, GENE, 36, 211-233, partly unpublished) was cut with restriction enzymes SalI and BglII. The DNA fragments were separated as described earlier and a 3200 bp piece from plasmid pWH102 and a 2900 bp piece of pPLcAT110 were ligated with the aid of T4-DNA ligase. After transformation into E. coli MC1061 (cI⁺) the correct plasmid containing transformant was screened as described above. The plasmid obtained was transformed to E. coli K-12 HI trp host. There is shown in Figure 7b a plasmid pCSS301 which is basically similar as pCSS112 except that instead of gene encoding bacterial luciferase there is a gene encoding green click beetle luciferase (Wood et al., 1989, Science, 244, 700-702). A plasmid pLucGR(tac) containing luciferase gene from click beetle was digested with restriction enzyme BspHI, the cohesive ends ends were made blunt by a Mung bean nuclease treatment and a DNA fragment of 1643 bp was separated on an agarose gel as described. This fragment was ligated to XbaI - BglII -digested vector pPLcAT110 (described earlier), which was filled in and CIP-treated. The ligation mixture was first transformed to E. coli MC1061 cells and after the correct plasmid was found from plasmid minipreparations it was transformed to E. coli K-12λHIλtrp. The symbols used are as in Figures 5, also p_{L} = leftward promoter of phage lambda., Pv = PvuI, Xb/Bs = ligation point XbaI, filled in, BsphI, Mung bean treated, Bs/Bg = ligation point BsphI, Mung bean treated, BglII, filled in.

A plasmid pCSS962 was constructed as follows: A shuttle vector p602/22, which can replicate both in E. coli and in B. subtilis (LeGrice et al., 1987, GENE, 55, 95-103) was cut with restriction enzyme BamHI, filled in with Klenow enzyme and treated with calf intestinal alkaline phosphatase (CIP). The plasmid pLucGR(tac) containing gene encoding green click beetle luciferase was digested with restriction enzyme BspHI, filled in with Klenow enzyme and separated on an agarose gel as described previously, was ligated to the above mentioned shuttle vector. The ligation mixture was transformed into E. coli MC1061 as will be described below and correct plasmid constructions were verified by analyzing the plasmid minipreparations with suitable restriction enzyme analysis. The correct plasmid was co-transformed with a helper plasmid pBL1 (LeGrice et al., 1987, GENE, 55, 95-103) in to B. subtilis 1A40 strain as will be described below. The plasmid and its construction is shown in Figure 7c. The symbols used are: P/O = Promoter/Operator; ori- = E. coli origin of replication; ori+ = B. subtilis origin of replication; kan = gene encoding kanamycin acetyltransferase; cat = gene encoding chloramphenicol acetyltransferase; T1 = transcriptional terminator.

### The competence induction of E. coli strains:

E. coli strains were made competent ie. able to take foreign DNA inside the cell as follows: E. coli was grown overnight in a volume of 5 ml in 2xTY medium and transferred to 100 ml of same medium. After about two hours the optical density as measured at 600 nm was 0.8. The cells were cooled on ice bath and centrifuged 4000xg for 5 min. The cell pellet was suspended in 50 ml of 50 mM CaCl₂ and centrifuged at 3000xg for 5 min at 0°C. The cells were suspended in 4 ml of 50 mM CaCl₂ containing glycerol 15 %. These competent cells were divided in 1 ml aliquotes and they were frozen rapidly in liquid nitrogen and stored at -70°C for later usage.

### The competence induction of B. subtilis:

B. subtilis 1A40 was grown overnight in 5 ml of 2xTY at 37°C, spun down and suspended in 15 ml of Growth Medium 1 [(SMS = (NH₄)₂SO₄ 0.2%, K₂HPO₄ 1.4%, KH₂PO4 0.6%, Na-citrate 0.1%, MgSO₄ 0.02%)), glucose 0.5%, casamino acids 0.05%, yeast extract 0.06%, MgCl₂ 1.5mM] and grown until the optical density was 1.8 as measured at 600 nm. The culture was then transferred to 150 ml of Growth Medium 2 (SMS, glucose 0.5%, casamino acids 0.01%, yeast extract 0.025%, MgCl₂ 5 mM, Ca(NO₃)₂) 2.5 mM for 90 minutes at 37°C. After centrifugation at 8000 rpm for 5 minutes at room temperature the pellet was suspended in 15 ml of the supernatant. Glycerol was added to 8% and cells were divided into 1 ml aliquotes which were quickly frozen using liquid N₂ and transferred to -70°C.

The transformation of E. coli strains with rec-DNA plasmids: Plasmid-DNA or ligation mixture was added to microcentrifuge tubes 1 to 10 µl on ice bath. To these 250 µl of competent-cells and 26 µl of 10xTMC (100 mM TRIS-HCl, pH 7.4, 100 mM MgCl₂, 100 mM CaCl₂) were added and kept at ice bath for 10 min with occasional careful mixing. A heat shock of two minutes at 42°C was given to cells and one ml of 2xTY was added. The cells were kept thereafter at 30°C for one hour and centrifuged 3 min 8000xg. The supernatant was discarded and cells were suspended in the leftover of supernatant (about 100 µl). The cell suspension was spread on antibiotic selection plates which were kept at 30°C overnight.

The transformation of B. subtilis with rec-DNA plasmids: One ml of frozen competent B. subtilis cells were quickly melted in a 37°C waterbath and they were diluted in 10 ml of SMS Dilution Medium (SMS, glucose 0.5%, MgCl₂ 20 mM, EDTA 1 mM). One ml of diluted cells were mixed with 1 ug of pCSS962 and 1 ug of pBL1 and incubated at 37°C for 30 minutes with shaking. After this cells were plated on 2xTY plates containing 10 ug/ml of kanamycin and erythromycin. Plates were incubated at 30°C for 22 hours.

### Example 1:

### The change in plasmid copy-number when cells are treated with nalidixic acid:

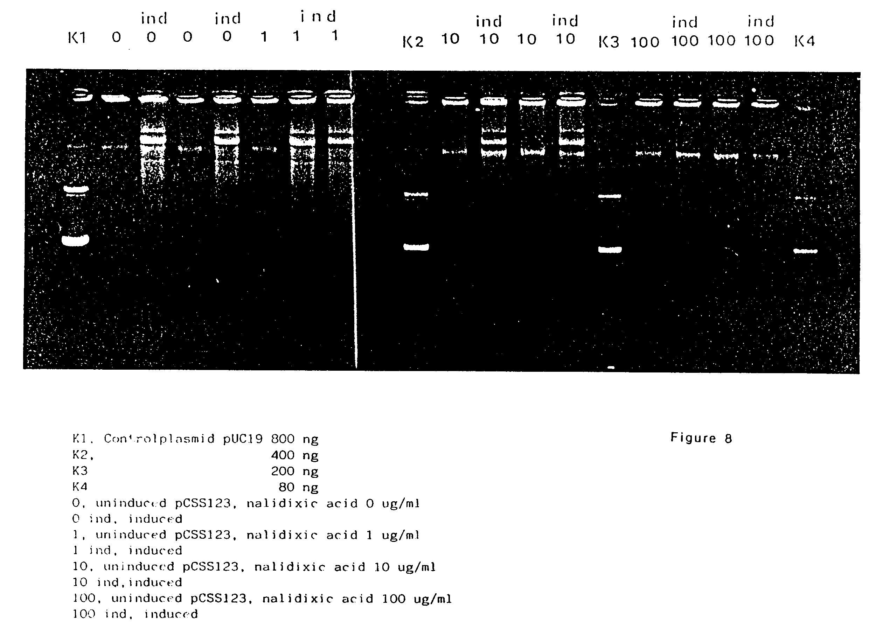

The plasmid pCSS123 described in the invention is a runaway-replication plasmid, in which the change in copy number can be obtained by shifting the temperature. This has been shown in Figure 8 where it is examined the amount and quality of plasmid-DNA extracted from heat-treated E. coli cells. The effect of an agent known to inhibit DNA replication, nalidixic acid, on cell DNA and especially on plasmid pCSS123 DNA is examined in the Figure.

E. coli pCSS123/JM103 cells were cultivated in 20 ml of 2xTY at 30°C in four Erlenmeyer bottles until the absorbance as measured at 600 nm was 0.3. Nalidixic acid, a known inhibitor of DNA replication, was added to the final concentration of 0, 1, 10 and 100 µg/ml. Immediately parallel samples of 1.5 ml from each bottle was withdrawn to 15 ml tubes and kept at 30°C for 20 min. The tubes were transferred to 42°C for one hour, the bottles were left to 30°C. After this both the tubes and the bottles were kept for an additional hour at 30°C in shaker, after which an extraction of total-DNA was performed from 1.5 ml of cultivations. Cells were centrifuged and the pellets were suspended in 500 µl 50 mM TRIS-HCl, pH 8.0, 50 mM EDTA. Cells were kept at ice bath for 30 min and 50 µl of lysozyme (10 mg/ml) was added and kept at ice bath for 45 min. One hundred µl of STEP solution (0.5% SDS, 50 mM TRIS-HCl, pH 7.5, 0.4 M EDTA) was added and kept at 50°C for 60 min. After this 600 µl of phenol was added and the tubes were gently mixed for 5 min and centrifuged 10 min at 12000xg. Two volumes of absolute ethanol and K-acetate, pH 6.0 to 0,3 M, were added to the supernatant to precipitate the DNA. After 30 min at -70°C the tubes were centrifuged 10 min at 12000xg and the pellet was washed with 500 µl of 70% ethanol, centrifuged and the pellets were dried in a vacuum exiccator for 5 min. The dried pellet was dissolved in 50 µl of 50 mM TRIS-HCl, pH 7.5, 1 mM EDTA, 100 µg/ml RNAase A solution and kept at 65°C for 20 min. A conventional agarose gel electrophoresis analysis was made for the extracted DNA's. This is shown in Figure 8 wherefrom one can see that those samples which where not heat-treated have a DNA content not much changing according to increased nalidixic acid concentration. On the other hand those samples which were heat-treated but did not contain nalidixic acid had a several fold increase in the amount of plasmid DNA. High amounts of nalidixic acid in the presence of cells either heat-treated or not did not change the plasmid content compared to the control. Already 1 ug nalidixic acid was enough to cause a clear decrease in the amount of plasmid-DNA in the heat-treated samples compared to untreated samples.

### Example 2:

### Determination of toxic substances as measured by the help of light production by cells containing plasmid whose copy-number can be changed.

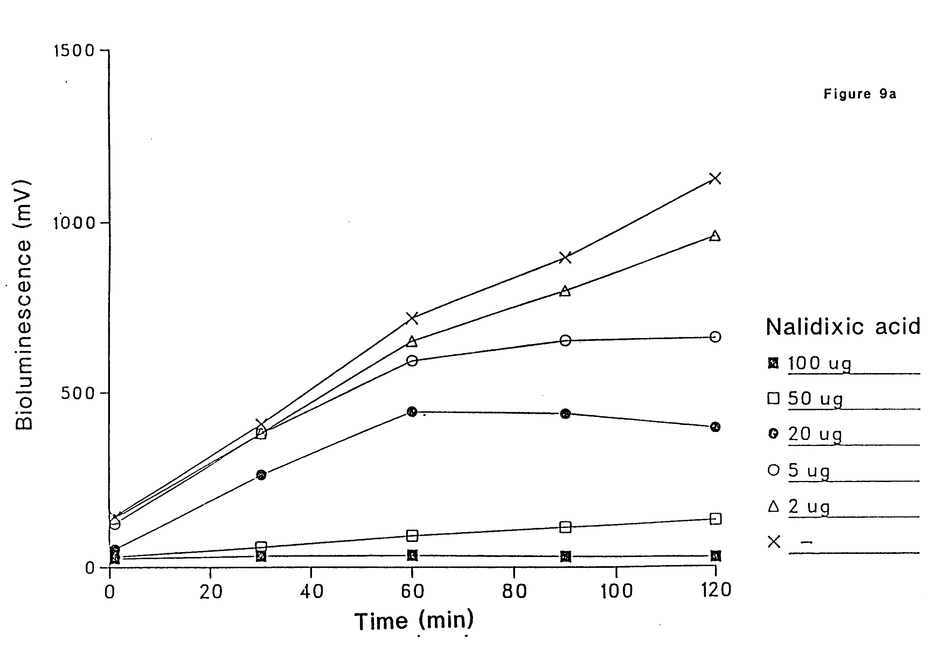

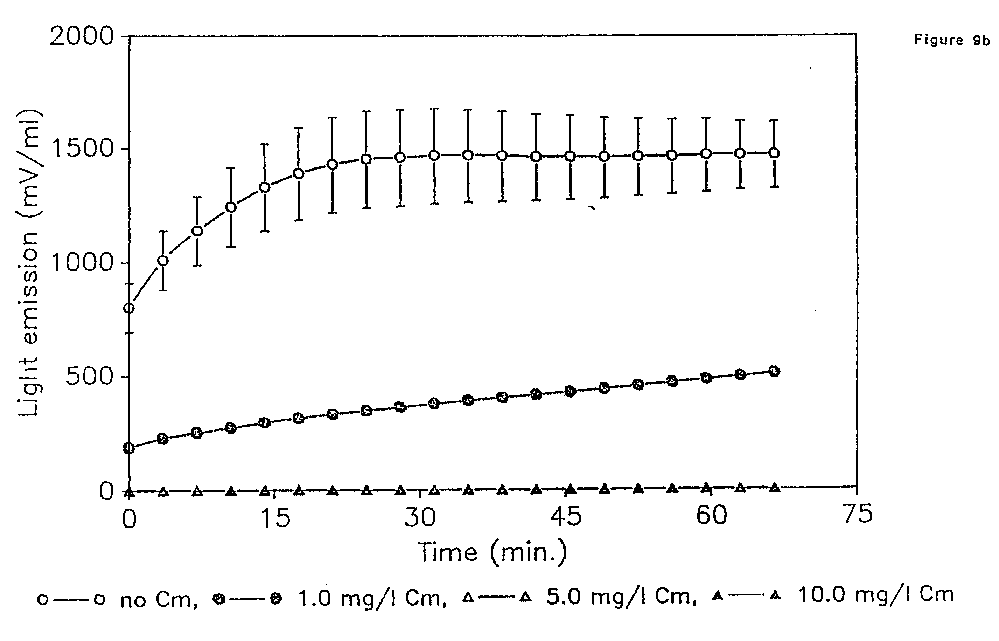

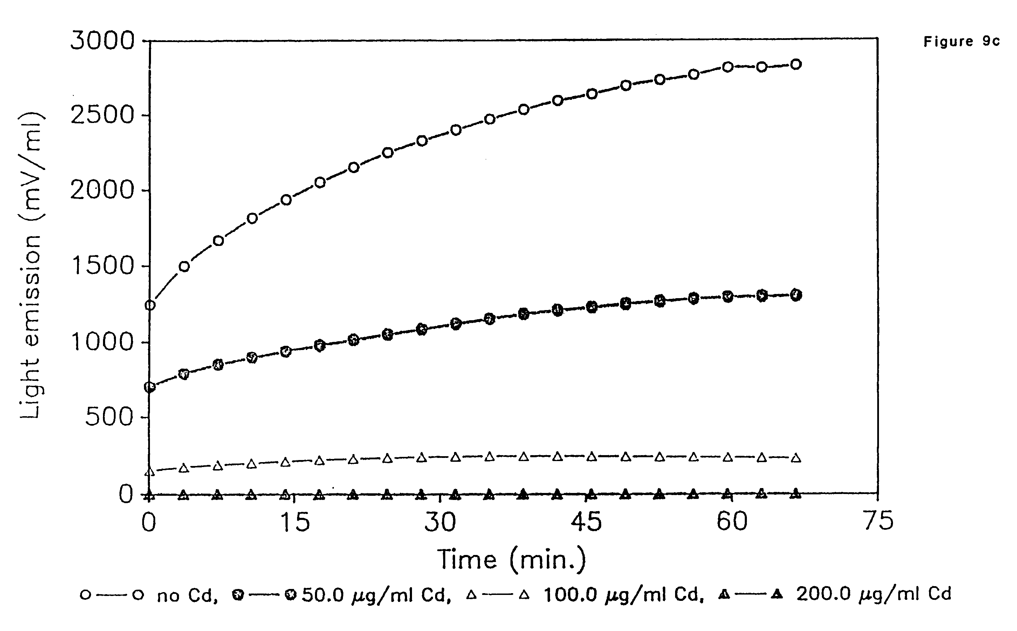

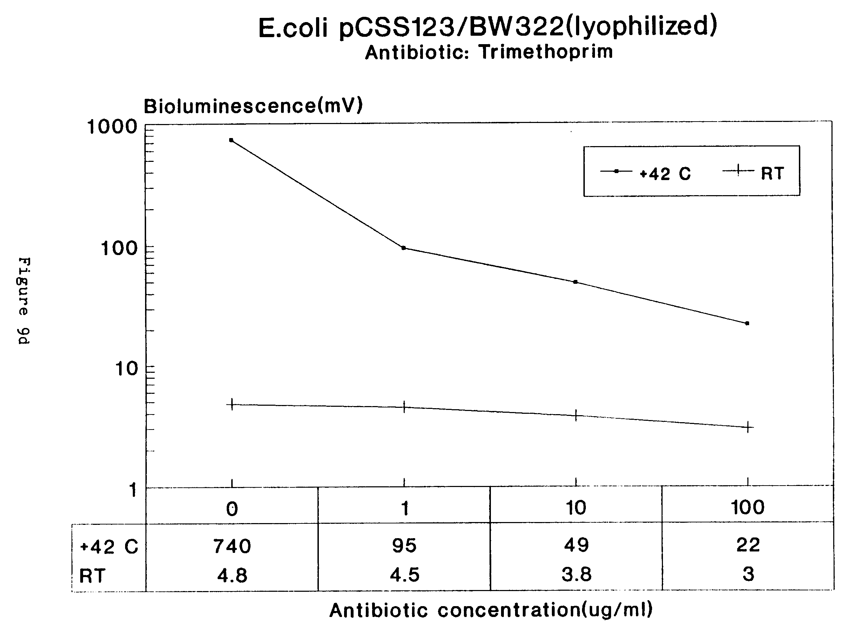

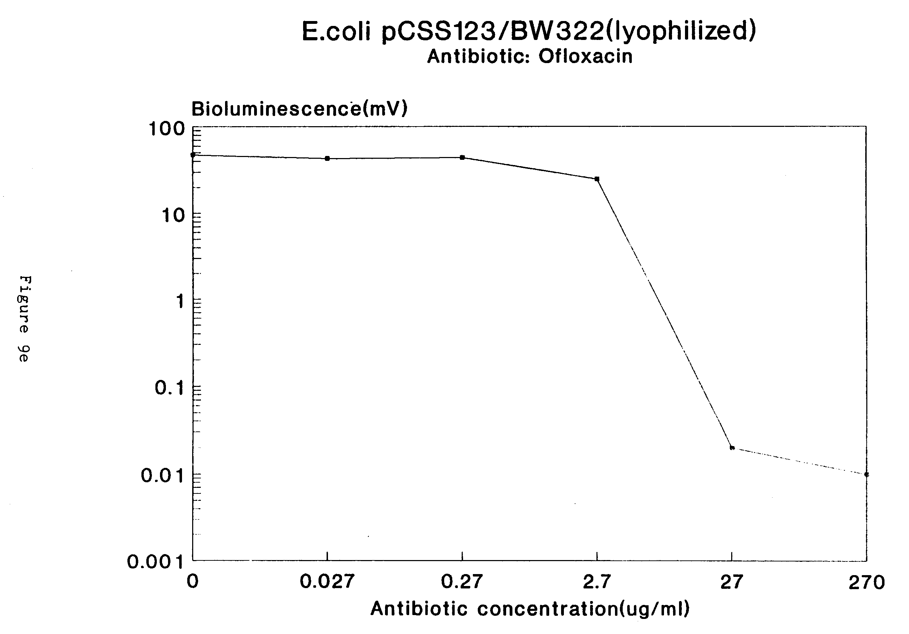

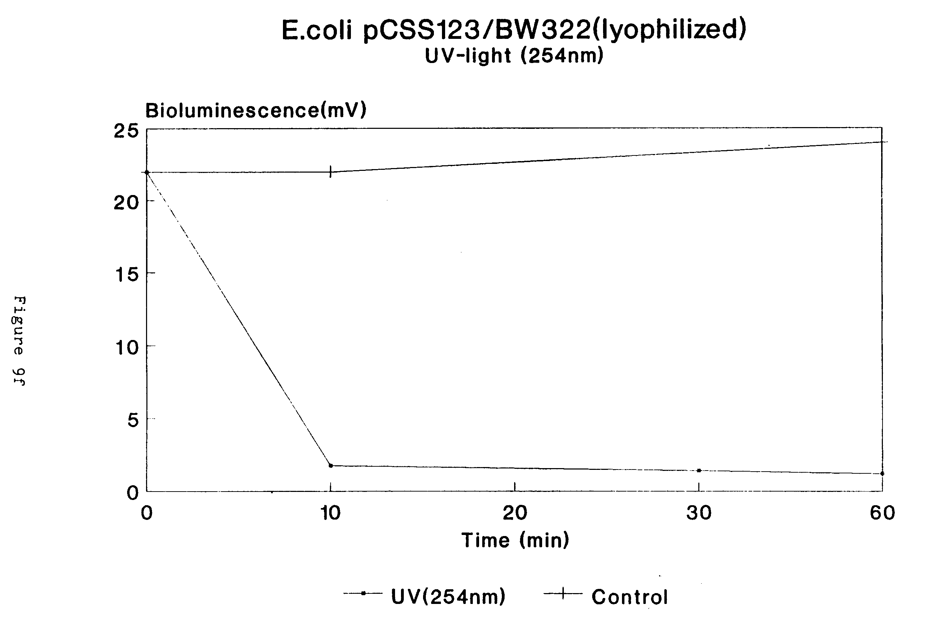

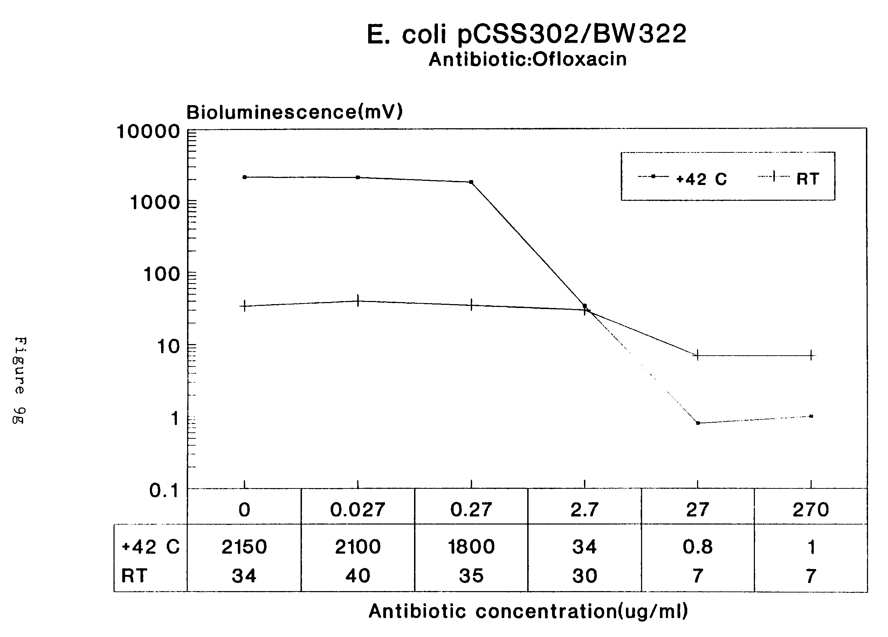

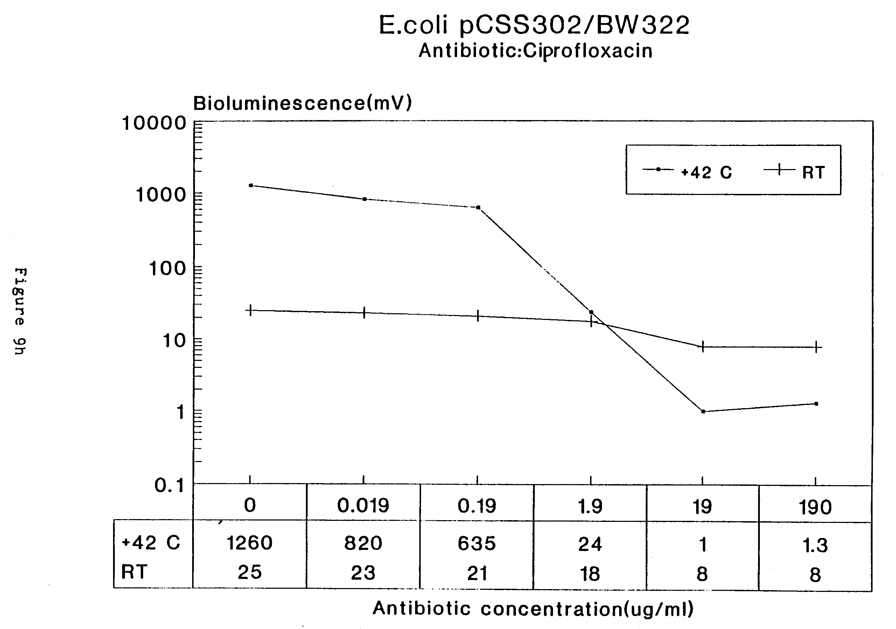

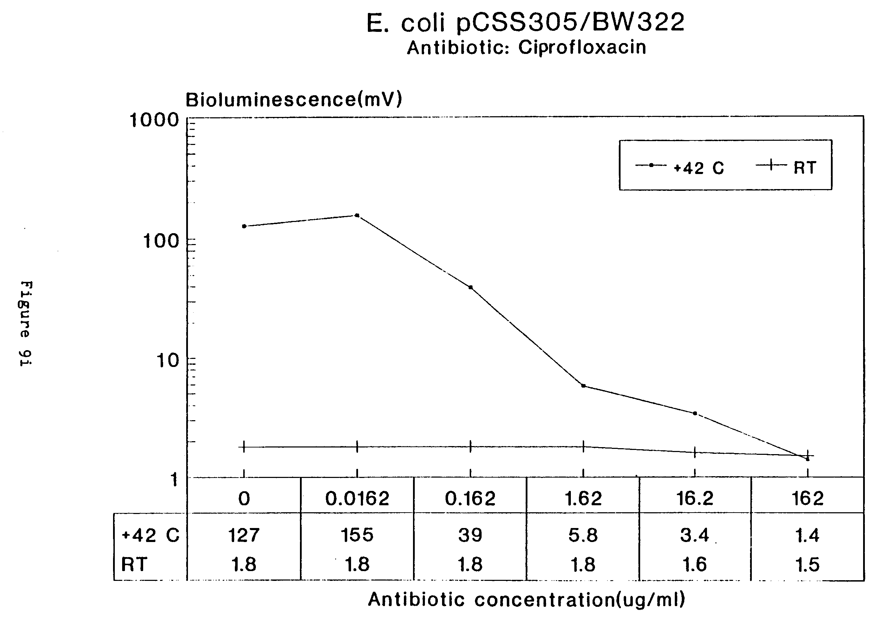

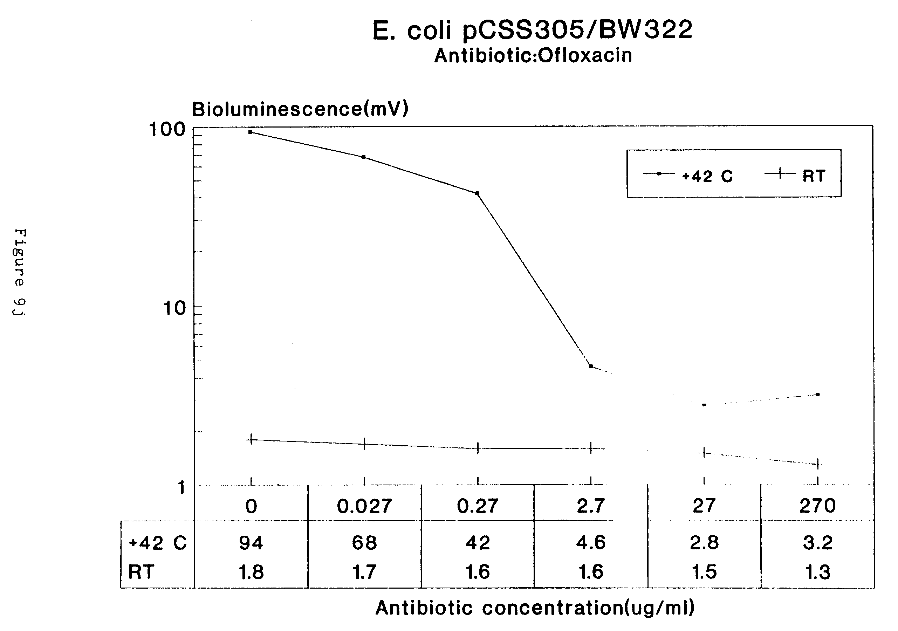

E. coli pCSS123/JM103 cells grown overnight were diluted 1:1000. Diluted cells in 2xTY were taken (0.5ml) and various amounts of antibiotics or other toxic substances were added. These solutions were incubated 20 min at 30°C. After this the samples were transferred to 42°C for one hour. Each tube was thereafter temperated to 30°C on waterbath and IPTG was added to 1 mM and n-decanal to 0.01 %. The tubes were transferred to an automated light-gathering device ie. luminometer 1251 (LKB-Wallac, Turku, Finland) whose measuring chamber had been temperated to 30°C. Measurement of light emission by the cells was done with the auto-mode program so that each tube was automatically measured in every two minutes. The data was collected in the memory of the computer for later analyses. There is shown in Figure 9a the detection of nalidixic acid using E. coli cells cloned with pCSS123. As can be seen from the Figure that already two µg's of nalidixic acid in the measuring conditions can be detected very fast. There is shown in Figure 9b the detection of chloramphenicol using the same approach as in Figure 9a. For clarity reasons only two concentrations of chloramphenicol was compared to the untreated control. There is shown in Figure 9c the detection of heavy metal cadmium using the same approach as in Figures 9a and 9b.

Freeze-dried E. coli pCSS123/BW322 were reconstituted with 1.0 ml of 2XTY and 45 µl of this was diluted 1:10 with 2xTY. Five µl of trimethoprim dilutions were added and kept at room temperature for 25 minutes. A heat induction to 42°C was for 25 minutes after which cells were temperated at 30°C water bath for 10 minutes and measured after the addition of n-decanal to 0.001% for light production with a LKB-Wallac 1250 manual luminometer. Same concentrations of trimethoprim together with reconstituted, freeze-dried cells which were not heat-treated acted as controls. As seen from Figure 9d that sensitivity of the assay is very high. There is shown in Figure 9e the detection of oflaxacin using the same approach as in Figure 9d.

100 µl of reconstituted E. coli pCSS123/BW322 was plated onto the Petri dish. Varying the time the effect of the UV light (254 nm) was tested. After this the samples were transferred to 42°C for 45 min and n-decanal to 0.01% was added. This is shown in Figure 9g.

E. coli pCSS302/BW322 cells grown overnight were diluted in 2xTY. 90 µl of the diluted cells was taken and the antibiotics were added. The solutions were incubated for 20 min at RT. After this the samples were transferred to 42°C for 45 min. The cells were measured for light production by adding 100 µl of solution containing 1 mM D-luciferin and 1mM ATP in 0.1M Na-citrate buffer, pH 5.0. In Figure 9g is shown the detection of oflaxacin and in Figure 9h is shown the detection of citrofloxacin.

E. coli pCSS305/BW322 cells were used to test the system where no substrate addition was needed to produce light from cells. To 90 µl of the cells in 2xTY 10 µl of the antibiotics was added. Different concentrations of oflaxacin (Figure 9j) and citrofloxacin (Figure 9i) were added and the tubes were kept at RT for 25 min. After this the induction was done by shifting the tubes to 42°C for 45 min. The tubes were loaded in the luminometer for light production measurement.

### Example 3:

### The detection of antibiotics with a method, where the control of plasmid replication is not possible:

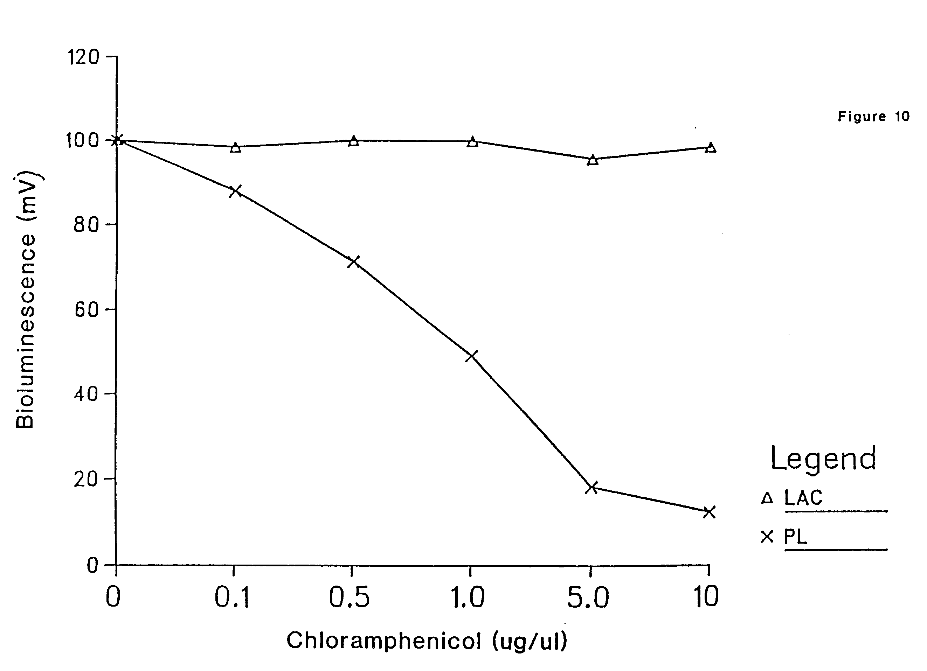

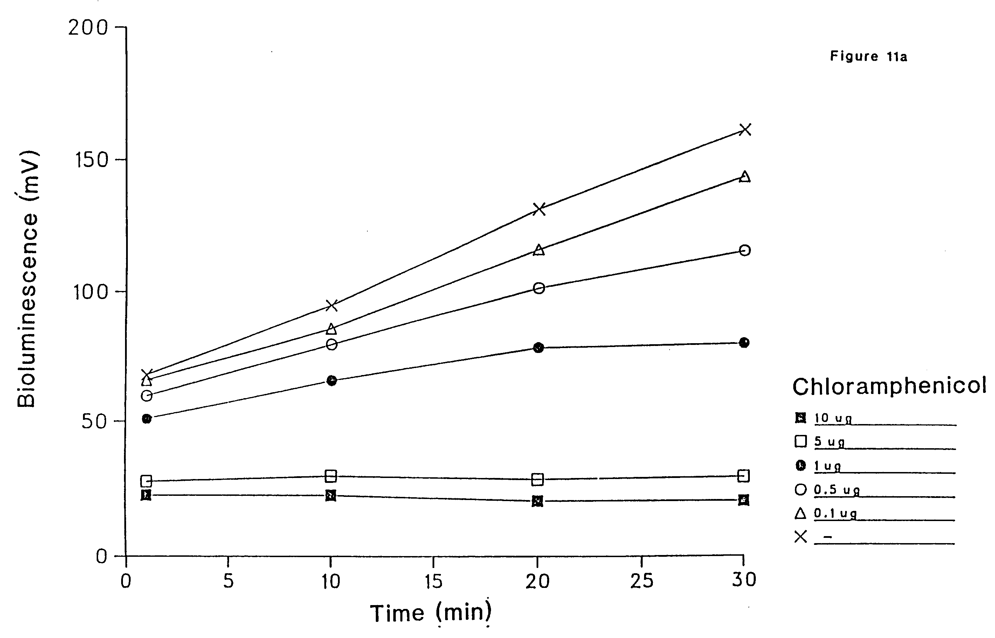

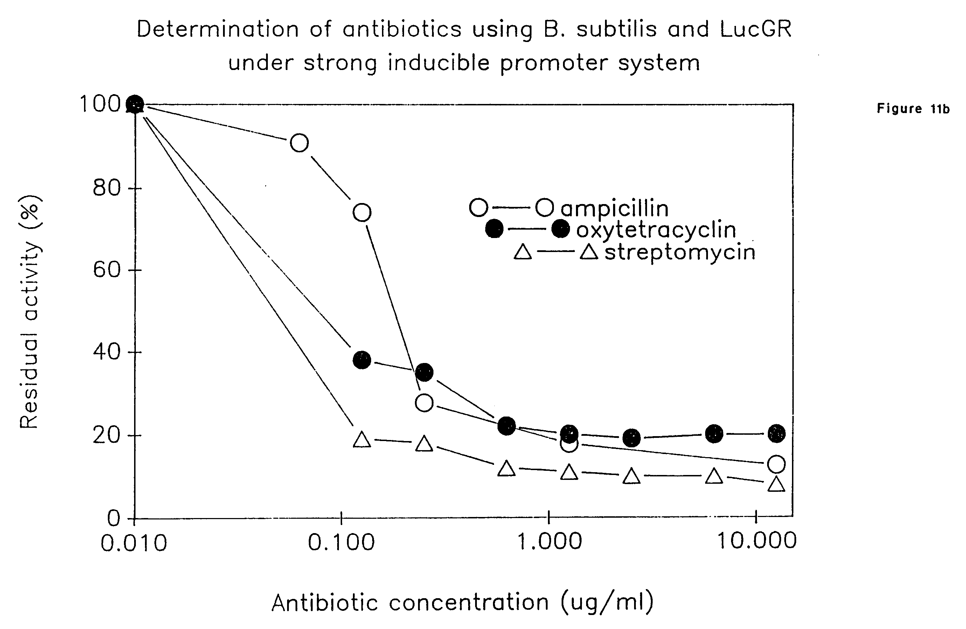

In the example a comparison is made between plasmids where the expression of bacterial luciferase genes are controlled by either lac (slow) or p_{L} promoter (fast). E. coli clones pCSS112/K-12λHIλtrp(cI857) were grown overnight in 2xTY medium containing ampicillin 100 µg/ml. After this a suitable dilution was made in HBSS-buffer or in milk and 500 µl of this was added to 3 ml luminometer tubes. The tubes were temperated at 30°C and different amounts of various toxic substances were added to tubes. Tubes were kept at 30°C for 20 min, after which the temperature was shifted to 42°C for 10 minutes. Thereafter the tubes were removed to luminometer chamber which had been temprated to 30°C for automated measurement. As a comparison an E. coli JM103 clone containing plasmid pCSS108 was used which was treated similarly without heat-shock step. In this case mRNA and protein synthesis were commenced by adding IPTG to 1 mM. It can be noticed from Figure 10, that when plasmid construction where p_{L} promoter directs the protein synthesis it is possible to detect toxic substance in much lower concentrations than using a slow and weaker lac promoter. In case of chloramphenicol the kinetics of light production has been shown in Figure 11a when plasmid pCSS112 in E. coli K-12λHIλtrp(cI857) strain is used. One can notice from the Figure that differences in the measured activity (light production) are seen from the start of measurement even in as low as 0.1 µg/ml concentrations in the measuring cuvette.

The detection of antibiotics belonging to penicillin family is of outmost importance since these antibiotics are very widely used and there do not exist fast methods to detect their presence. There is shown in Figure 11b the determination of ampicillin and also oxytetracyclin and streptomycin using B. subtilis 1A40 cells cloned with luciferase gene from a click beetle. The plasmid used in measurement is shown in Figure 7c. Using this construction the production of luciferase can be turned on in B. subtilis by simple addition of IPTG, which binds to the lac repressor coded by the helper plasmid pBL1 present in the same cell. After binding to the repressor it is not any more able to bind to the DNA region between phage T5 promoter and the luciferase gene thus allowing the expression of luciferase. Cells containing both plasmids were cultivated overnight in 2xTY containing erythromycin (10 µg/ml, to keep pBL1 in the cell) and kanamycin (10 µg/ml, to keep pCSS962 in the cell). A suitable dilution was made and different amounts of antibiotic was added to the cells. After an incubation period of 2 hours at 30°C the tubes were measured for light emission after addition of 1 mM D-luciferin substrate in 0.1 M Na-citrate. As can be seen from Figure as low amount of 0.1 ug/ml of ampicillin and even lower amounts of oxytetracyclin and streptomycin can be detected.

### Example 4:

### Detection of toxic substances using a method where E. coli contains constant copy-number rec-DNA plasmid and in which p_{L} promoter of phage lambda controls the biosynthesis of bacterial luciferase and click beetle luciferase:

In the following there is shown some examples on detection of substances affecting other biosynthetic routes and metabolism of cells. The tests have been performed in the same way as those described in previous examples. The goal has been to develop an extremely rapid method, which would anyhow be also very sensitive. Plasmid pCSS112 cloned in the E. coli K-12 strain was used throughout in the following examples.

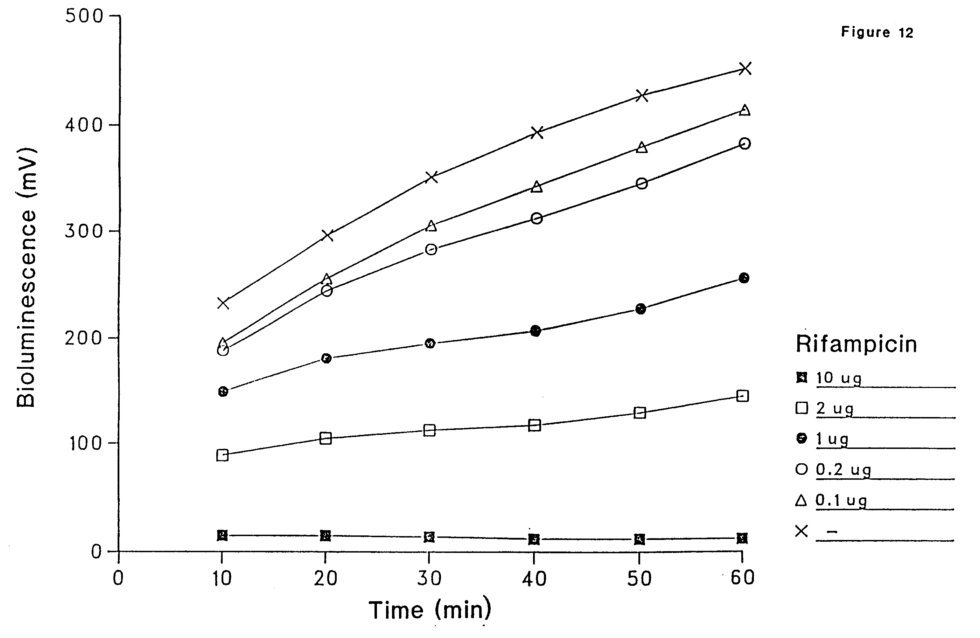

The following figures show the effect of each tested substance as measured by light production. As in earlier measurements the presence of inhibiting factors is seen as lowered light production compared to cases where factor has not been present. There is shown in Figure 12 the detection of an antibiotic, rifampicin, which is a known inhibitor of transcription ie. formation of messenger RNA.

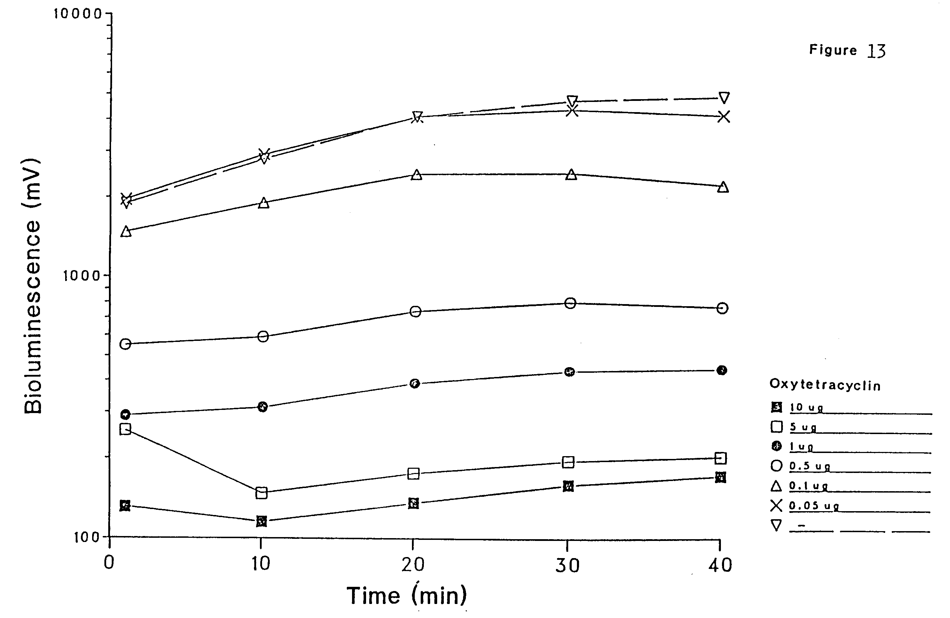

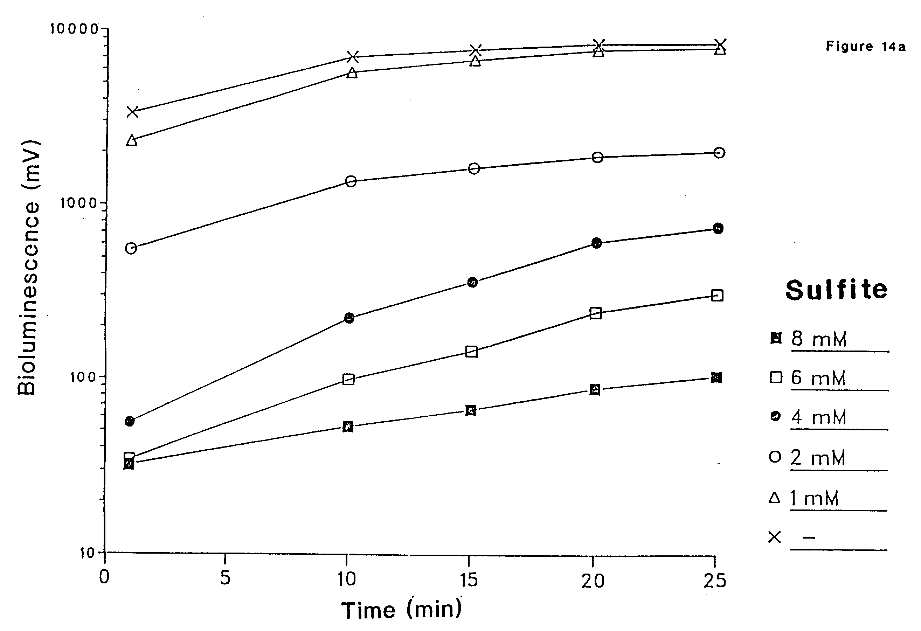

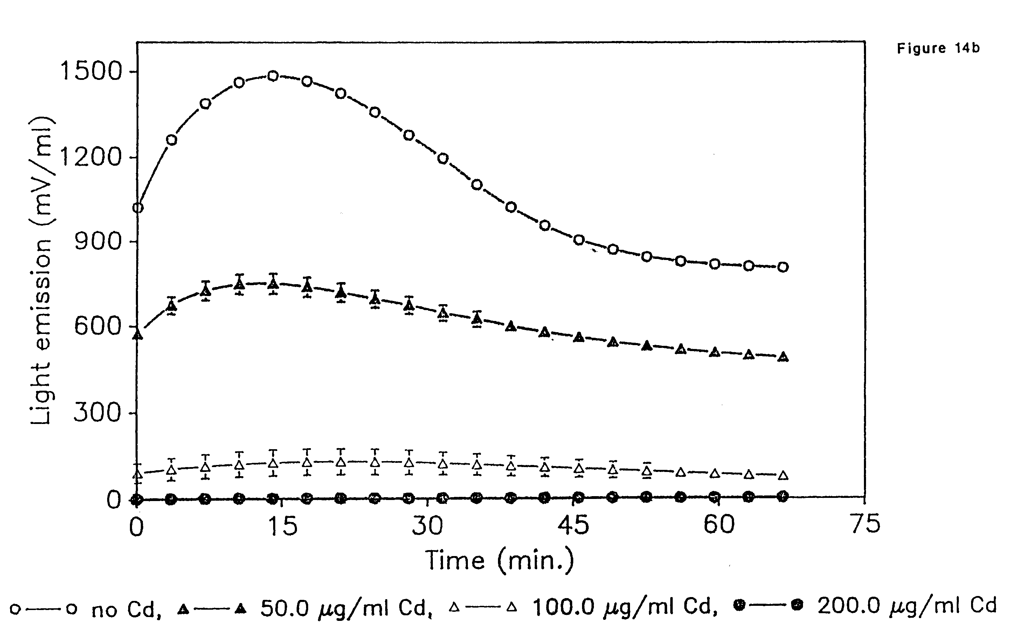

As low amount as one µg can be seen very rapidly with the test described in this invention. The effect of oxytetracyclin which binds to 30S ribosomal subunit on the measuring system described here is shown in Figure 13. As in the rifampicin case also here the effect of this antibiotic is strong and easily detected. The effect of sulphite which is a known inhibitor of metabolism and very much used in food processing is shown in Figure 14a. The effect of heavy metal cadmium which is also a known inhibitor of metabolism contaminating soils and water is shown in Figure 14b. These results show that the test system described in this invention is also applicable to the fast determination of metabolic inhibitors. Also it shows that the method can detect the presence of also other agents than antibiotics.

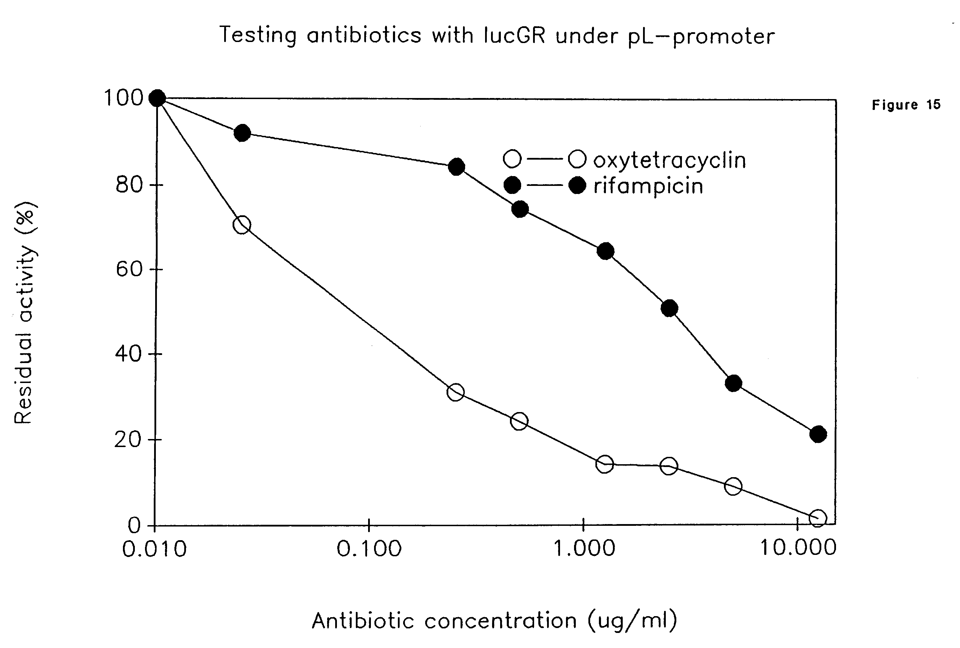

One is able to use different luciferases instead of bacterial luciferase from V. harveyi without losing sensitivity or other performance of the test. There is shown in Figure 15 an analogous measurement as shown in Figures 13 and 14. In the plasmid used in this test (pCSS301) the bacterial luciferase was compensated with click beetle luciferase as described in Figure 7b. The test was done essentially as with bacterial luciferase except that after the cells had been incubated with or without toxic substances 10 minutes at 42°C the cells were measured for light production after 15 minutes temperation time at 30°C by adding 100 µl of solution containing 1 mM D-luciferin, 1 mM ATP in 0.1 M Na-citrate buffer, pH 5.0. Thereafter the light production was measured using a manual luminometer 1250 (LKB-Wallac, Turku, Finland). As can be seen from the Figure the sensitivity of the method to detect either oxytetracycline or rifampicin is extremely high and comparable to the detection made with bacterial luciferase.

### Example 5:

### The determination of a toxic substance using a method where p_{L} promoter of phage lambda activates the biosynthetic machinery to produce β-galactosidase:

In the previous examples the basis for measurements was the light produced by the bacteria, which phenomenon was due to genes encoding luciferase. As a protein one is able to use whatever protein or peptide, for which there is a method to measure. In this example the gene encoding luciferase has been changed to a gene encoding β-galactosidase of E. coli. The plasmid pPLcAT14 used has been described earlier (Stanssens, P., Remaut, E. & Fiers, W., 1985, GENE, 36, 211-223).

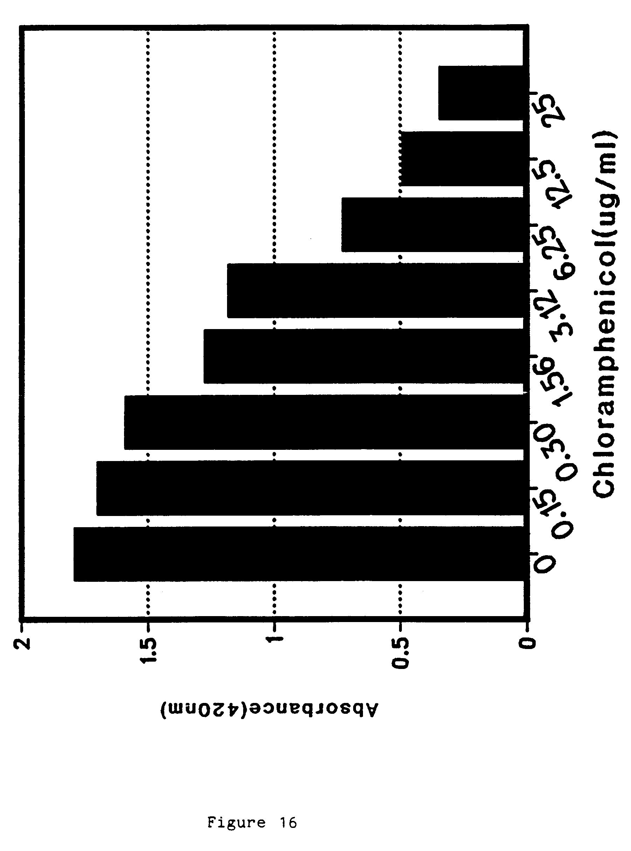

E. coli clones pPLcAT14/K-12λHIλtrp were grown overnight in 2xTY medium which was suplemented with ampicillin 100 µg/ml. After this a suitable dilution was made from bacteria in HBSS buffer and 80 µl of this was added to glas tubes. Different concentrations of chloramphenicol was added and tubes were kept at room temperature for 15 minutes. After incubation the activation of p_{L} promoter was performed by shifting the tubes to 42°C for 30 minutes. As a concequence the biosynthesis machinery is activated to produce β-galactosidase coded by the β-galactosidase gene cloned under p_{L} promoter in plasmid pPLcAT14. After induction toluene is added to tubes to 10 % which makes the cells porous to ONPG chemical. β-galactosidase forms yellow colour which can be measured with a spectrophotometer at 420 nm after reaction with ONPG. After this tubes were centrifuged and measured at 420 nm. There is shown the effect of chloramphenicol in Figure 16 using our method where the protein to be detectd was β-galactosidase and its substrate produce a coloured product.

### Example 6:

### Determination of organic content of solution using a method where p_{L} promoter of phage lambda activates the biosynthetic machinery to produce luciferase:

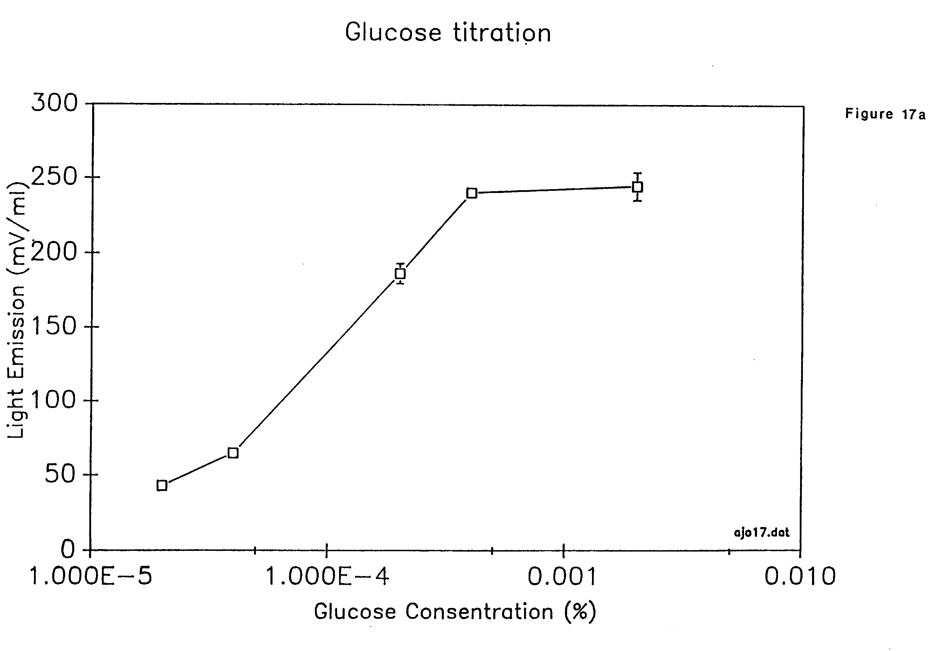

E. coli pCSS112/K-12λHIλtrp cells were cultivated overnight in 2xTY containing ampicillin (100 µg/ml). Cells were spun down and washed twice with a HBSS medium (Korpela & Karp, 1988, Biotech. Lett., 10, 383-388) omitting glucose and gelatine but supplemented with tryptophane 0.02%. The cells were shaken at this medium for further 4 hours, spun down and suspended in HBSS buffer containing either 0.1% glucose or 0.1% (NH₄)₂SO₄ depending on whether carbon sources or nitrogen sources were evaluated, respectively. A suitable dilution was made from treated cells in minimal salts, various amounts of either carbon or nitrogen sources were added and the cells were incubated 10 minutes at 30°C. A heat treatment of 10 minutes at 42°C was given to cells to start the protein synthesis after which the cells were temperated 10 minutes at 30°C before the tubes were loaded in the automated luminometer for light production measurements after addition of n-decanal to 0.001%. There is shown in Figure 17a the detection of glucose.

**Table 1**

| Biochemical targets for drug action: | | |
|---|---|---|
| Cell walls | Inhibitors of protein synthesis | Inhibitors of nucleic acid synthesis |
| beta-lactams | chloramphenicol | nalidixic acid |
| cephalosporins | tetracyclines | novobiocin |
| bacitracin | aminoglycosides | rifamycins |
| vancomycin | macrolides | phleomycin |
| polymyxins | erythromycin | mithramycin |
| gramicidins | lincomycin | actinomycin |
| valinomycin | puromycin | quinolones |

## Claims

1. A method for determining the presence or amount of a factor in a sample to be tested, wherein the factor affects directly or indirectly the DNA, RNA and/or proteins of the cell or the synthesis mechanisms thereof, **characterized** in
a) incubating a sample to be tested with a population of transformed cells for a period sufficient to allow said factor, if present in the sample, to affect said cells, said cells being transformed with a recombinant-DNA plasmid, the replication of said plasmid being subject to a regulatable promoter, which can be induced by an exogenous stimulus independent of replication of the cells;
b) then applying said exogenous stimulus to said cells to induce replication of said plasmid, whereupon the copy number of the plasmid will begin to increase in the cell, if the factor has not affected the plasmid by inhibiting the replication; and
c) the change in the copy number of the recombinant-DNA plasmid in the cell is determined directly or indirectly, and compared with a reference test in which the factor was not present or in which it was present in a known amount, and thereby the presence or the amount of the factor is determined.

2. A method according to claim 1, **characterized** in that the recombinant-DNA plasmid contains a DNA sequence which encodes at least one selected protein or a part of it that is essential for biological activity of said protein, and the recombinant DNA plasmid may comprise one or more DNA sequences which make the cell resistant to an antibiotic, heavy metal, or toxin.

3. A method according to claim 2, **characterized** in that the DNA sequence encoding the protein is subject to a regulatable promoter which is controlled by positive or negative feedback and is regulatable in said cells, and is activated simultaneously or at a desired moment subsequent to induction of plasmid replication.

4. A method according ot claim 3, **characterized** in that the factor to be determined is a factor affecting the biosynthesis or replication of the recombinant DNA plasmid, the biosynthesis of the RNA, transcription, translation, the cell membranes, metabolism, or enzyme activity.

5. A method according to claim 4, **characterized** in that the factor is aflatoxin, heavy metal, ethidium bromide, nalidixic acid, trimethoprim, fluoroquinolone, aminoglycoside, penicillin, cephalosporin, rifampicin, chloramphenicol, tetracycline, sulphonamide, and the cell used is sensitive to the factor to be determined.

6. A method according to claim 4 or 5, **characterized** in that the cell is Escherichia coli and the mechanism resposible for the replication of the recombinant DNA plasmid contained in the bacteria can be regulated with precision by means of a strong promoter, such as lambda P_{L} and P_{R} promoters, lac, trp, various hybrid promoters such as tac and artificial promoters.

7. A method according to claim 6, **characterized** in that the recombinant DNA plasmid is pCSS123, pCSS302 or pCSS305.

8. A method for determining the presence or amount of a factor in a sample to be tested, wherein the factor affects directly or indirectly the DNA, RNA and/or proteins of the cell or the synthesis mechanisms thereof, **characterized** in
a) incubatiang a sample to be tested with a population of transformed cells for a period sufficient to allow said factor, if present in the sample, to affect said cells, said cells being transformed with a high copy number recombinant DNA plasmid containing a DNA sequence encoding a marker protein or a part thereof essential to the biological activity of said protein, said sequence being coupled to a regulatable promoter such that expression of said marker protein is inducible by an exogenous stimulus;
b) then applying said exogenous stimulus to said population of cells to induce expression of said marker protein, whereby the amount of the marker protein in the cell starts to grow, if the factor has not affected the protein synthesis directly or indirectly;
c) determining the amount of the marker protein expressed by said population of cells, and comparing said amount with a reference test in which the factor was not present or in which it was present in a known amount, and thereby the presence or the amount of the factor is determined.

9. A method according to claim 1 or 8, **characterized** in that the cell is a gram-negative or gram-positive bacterium such as one of the Enterobacteriaceae group, preferably Escherichia coli, or such as one of the Bacillus group, preferably Bacillus subtilis.

10. A method according to claims 1-3, 8 or 9, **characterized** in that the exogenous stimulus is produced either from the chromosomal DNA of the host cell, a plasmid in the cell belonging to another incompatibility class, the same plasmid, a lytic or lysogenic phage or virus, or the exogenous stimulus is added from outside the cells, for example by adding chemical compounds, altering the temperature, or by radiation.

11. A method according to claim 10, **characterized** in that the recombinant DNA plasmid can comprise one or more DNA sequences which make the cell resistant to antibiotics, heavy metals or toxins.

12. A method according to claim 1 or 8, **characterized** in that the examined sample is an aerosol in a gaseous, liquid or solid form, or the sample is radioactive, ultraviolet or other radiation, and of a biological or non-biological origin.

13. A method according to claim 1 or 8, **characterized** in that the cells are lyophilized and rehydrated before the determination by a suitable liquid or cultivation medium.

14. A method according to claim 8, **characterized** in that the factor is a factor affecting the biosynthesis of the DNA, the biosynthesis of the RNA, transcription, translation, cell membranes or metabolism of the cell containing said recombinant plasmid, such as mutagen, antibiotic, heavy metal or toxin.

15. A method according to claim 14, **characterized** in that the cell is Bacillus subtilis.

16. A method according to claim 15, **characterized** in that the marker protein in the recombinant DNA plasmid is subject to a regulatable strong promoter such as Φ 105, phage T5 promoter controlled by lac operator, or saccharose regulatable promoter, and that the marker protein is alpha-amylase, alkaline phosphatase, β-galactosidase, luciferase, peroxidase, T4 lysozyme, β-glucuronidase, oxidoreductase or pyrophosphatase.

17. A method according to claim 14, **characterized** in that the cell is Escherichia coli.

18. A method according to claim 6 or 17, **characterized** in that the recombinant DNA plasmid comprises a DNA sequence encoding the protein of a virus, a procaryotic cell or a eucaryotic cell or its part essential with regard to its biological activity, and the expression of the protein is controllable by means of a regulatable promoter, such as lac, trp, P_{L} and P_{R}, various hybrid promoters, such as tac, or artificial promoters.

19. A method according to claim 18, **characterized** in that the protein is luciferase, β-galactosidase, alkaline phosphatase, peroxidase, T4 lysozyme, β-glucuronidase, oxidoreductase or pyrophosphatase.

20. A method according ot claim 16 or 19, **characterized** in that the recombinant DNA plasmid encodes the luciferase of Vibrio harveyi or another bacterial luciferase, or beetle luciferase.

21. A method according to claim 20, **characterized** in that the recombinant-DNA plasmid is plasmid pCSS112, pCSS301 or pCSS962.

22. A method according to any of claims 19-21, **characterized** in that aldehyde or luciferin is added to the reaction when measuring the amount/activity of expressed luciferase in the bacteria.

23. A method according to claim 4, 5 or 14, **characterized** in that the determination is made of milk, serum or water.

## Patentansprüche

1. Verfahren zum Nachweis oder zur Bestimmung der Menge eines Faktors in einer zu untersuchenden Probe, wobei der Faktor die DNA, RNA und/oder Zellproteine oder deren Synthesemechanismen direkt oder indirekt beeinträchtigen kann,
gekennzeichnet durch
a) Inkubation einer zu untersuchenden Probe mit einer Population aus transformierten Zellen für eine Dauer, die für den genannten Faktor, wenn er in der Probe vorhanden ist, ausreicht, um die genannten Zellen zu beeinträchtigen, wobei die genannten Zellen durch ein rekombinantes DNA-Plasmid transformiert werden und die Replikation des genannten Plasmids einem regulierbaren Promotor unterworfen ist, der durch einen exogenen Reiz unabhängig von der Replikation der Zellen induziert werden kann;
b) nachfolgendes Einwirken des genannten exogenen Reizes auf die Zellen, um die Replikation des genannten Plasmids zu induzieren, worauf die Kopienzahl des Plasmids in der Zelle anzusteigen beginnt, sofern der Faktor das Plasmid nicht durch Hemmung der Replikation beeinträchtigt hat ; und
c) direkte oder indirekte Bestimmung der Veränderung der Kopienzahl des rekombinanten DNA-Plasmids in der Zelle und Vergleich mit einem Vergleichstest, bei dem der Faktor nicht vorhanden oder bei dem er in bekannter Menge vorhanden war, wodurch der Nachweis des Faktors erbracht oder die Menge des Faktors bestimmt wird.

2. Verfahren nach Anspruch 1,
dadurch gekennzeichnet,
daß das rekombinante DNA-Plasmid eine DNA-Sequenz aufweist, die zumindest für ein ausgewähltes Protein oder einen Teil desselben kodiert, der unentbehrlich für die biologische Aktivität des genannten Proteins ist, und wobei das rekombinante DNA-Plasmid eine oder mehrere DNA-Sequenzen umfassen kann, die die Zelle resistent gegenüber einem Antibiotikum, einem Schwermetall oder einem Toxin machen.

3. Verfahren nach Anspruch 2,
dadurch gekennzeichnet,
daß die für das Protein kodierende DNA-Sequenz einem regulierbaren Promotor unterworfen ist, der durch positive oder negative Rückkopplung kontrolliert wird und in den genannten Zellen regulierbar ist und gleichzeitig mit oder zu einem beliebigen Zeitpunkt nach der Einleitung der Plasmidreplikation aktiviert wird.

4. Verfahren nach Anspruch 3,
dadurch gekennzeichnet,
daß der zu bestimmende Faktor ein Faktor ist, der die Biosynthese oder die Replikation des rekombinanten DNA-Plasmids, die Biosynthese der RNA, die Transkription, die Translation, die Zellmembranen, den Metabolismus oder die Enzymaktivität beeinträchtigt.

5. Verfahren nach Anspruch 4,
dadurch gekennzeichnet,
daß der Faktor Aflatoxin, Schwermetall, Ethidiumbromid, Nalidixinsäure, Trimethoprim, Fluoroquinolon, Aminoglycosid, Penicillin, Cephalosporin, Rifampicin, Chloramphenicol, Tetracyclin, Sulfonamid ist und die verwendete Zelle gegenüber dem zu bestimmenden Faktor empfindlich ist.

6. Verfahren nach Anspruch 4 oder 5,
dadurch gekennzeichnet,
daß die Zelle Escherichia coli ist und der in den Bakterien enthaltene Mechanismus, der für die Replikation des rekombinanten DNA-Plasmids verantwortlich ist, präzise mit Hilfe eines starken Promotors, wie z. B. lambda P_{L} und P_{R} Promotoren, lac, trp, verschiedene Hybridpromotoren wie z. B. tac und künstliche Promotoren reguliert werden kann.

7. Verfahren nach Anspruch 6,
dadurch gekennzeichnet,
daß das rekombinante DNA-Plasmid pCSS123, pCSS302 oder pCSS305 ist.

8. Verfahren zum Nachweis oder zur Mengenbestimmung eines Faktors in einer zu untersuchenden Probe, wobei der Faktor direkt oder indirekt die DNA, RNA und/oder Proteine der Zelle oder deren Synthesemechanismen beeinträchtigt,
gekennzeichnet durch
a) Inkubation einer zu untersuchenden Probe mit einer Population transformierter Zellen für eine Dauer, die für den genannten Faktor, wenn er in der Probe vorhanden ist, ausreicht, um die genannten Zellen zu beeinträchtigen, wobei die genannten Zellen durch ein rekombinantes DNA-Plasmid mit hoher Kopienzahl transformiert werden, das eine DNA-Sequenz umfaßt, die für ein Markerprotein oder für einen Teil davon kodiert, der für die biologische Aktivität des Proteins unentbehrlich ist, wobei diese Sequenz mit einem regulierbaren Promotor gekoppelt ist, so daß die Expression des genannten Markerproteins durch einen exogenen Reiz induzierbar ist;
b) nachfolgende Einwirkung des genannten exogenen Reizes auf die genannte Zellpopulation, um die Expression des genannten Markerproteins zu induzieren, wobei die Menge des Markerproteins in der Zelle anwächst, wenn der Faktor die Proteinsynthese nicht direkt oder indirekt beeinflußt hat;
c) Bestimmung des von der genannten Zellpopulation exprimierten Markerproteins und Vergleich mit einem Vergleichstest, bei dem der Faktor nicht vorhanden oder bei dem er in bekannter Menge vorhanden war, wodurch der Nachweis des Faktors erbracht oder die Menge des Faktors bestimmt wird.

9. Verfahren nach Anspruch 1 oder 8,
dadurch gekennzeichnet,
daß die Zelle ein gramnegatives oder grampositives Bakterium ist, z. B. eines der Enterobacteriaceae-Gruppe, vorzugsweise Escherichia coli, oder eines der Bacillus-Gruppe,
vorzugsweise Bacillus subtilis.

10. Verfahren nach einem der Ansprüche 1 bis 3, 8 oder 9,
dadurch gekennzeichnet,
daß der exogene Reiz entweder von der chromosomalen DNA der Wirtszelle, einem Plasmid der Zelle, das zu einer anderen Inkompatibilitätsklasse gehört, dem gleichen Plasmid, einem lytischen oder lysogenen Phagen oder Virus ausgeht, oder der exogene Reiz wird von außerhalb der Zellen zugeführt wird, zum Beispiel durch Zugabe von chemischen Verbindungen, Temperaturänderungen oder durch Strahlung.

11. Verfahren nach Anspruch 10,
dadurch gekennzeichnet,
daß das rekombinante DNA-Plasmid eine oder mehrere DNA-Sequenzen aufweisen kann, die die Zelle gegenüber Antibiotika, Schwermetallen oder Toxinen resistent machen.

12. Verfahren nach Anspruch 1 oder 8,
dadurch gekennzeichnet,
daß die untersuchte Probe ein Aerosol mit gasförmigen, flüssigen oder festen Bestandteilen ist, oder die Probe radioaktiv, ultraviolett oder anderweitig strahlend ist und biologischen oder nicht-biologischen Ursprungs ist.

13. Verfahren nach Anspruch 1 oder 8
dadurch gekennzeichnet,
daß die Zellen vor der Bestimmung in einer geeigneten Flüssigkeit oder Kulturmedium gefriergetrocknet und rehydratisiert werden.

14. Verfahren nach Anspruch 8,
dadurch gekennzeichnet,
daß der Faktor ein Faktor ist, der die Biosynthese der DNA, die Biosynthese der RNA, die Transkription, die Translation, die Zellmembranen oder den Metabolismus der Zelle beeinflußt, die das genannte rekombinante Plasmid enthält, wie z. B. ein Mutagen, ein Antibiotikum, ein Schwermetall oder ein Toxin.

15. Verfahren nach Anspruch 14,
dadurch gekennzeichnet,
daß die Zelle ein Bacillus subtilis ist.

16. Verfahren nach Anspruch 15,
dadurch gekennzeichnet,
daß das Markerprotein im rekombinanten DNA-Plasmid einem regulierbaren starken Promotor unterworfen ist, wie z. B. Φ 105, Phage T5-Promotor, der von einem lac-Operator kontrolliert wird, oder ein Saccharose-regulierbarer Promotor, und daß das Markerprotein Alpha-Amylase, alkalische Phosphatase, β-Galactosidase, Luciferase, Peroxidase, T4-Lysozym, β-Glucuronidase, Oxidoreductase oder Pyrophosphatase ist.

17. Verfahren nach Anspruch 14,
dadurch gekennzeichnet,
daß die Zelle Escherichia coli ist.

18. Verfahren nach Anspruch 6 oder 17,
dadurch gekennzeichnet,
daß das rekombinante DNA-Plasmid eine DNA-Sequenz umfaßt, die für das Protein eines Virus, einer prokariotischen Zelle oder einer eukaryotischen Zelle kodiert oder deren für die biologische Aktivität unentbehrlichen Teil, und daß die Expression des Proteins mit Hilfe eines regulierbaren Promotors kontrolliert werden kann, wie z. B. lac-, trp-, P_{L}- und P_{R}-Promotoren, verschiedene Hybridpromotoren wie z. B. tac oder künstliche Promotoren.

19. Verfahren nach Anspruch 18,
dadurch gekennzeichnet,
daß das Protein Luciferase, β-Galactosidase, alkalische Phosphatase, Peroxidase, T4-Lysozym, β-Glucuronidase, Oxidoreductase oder Pyrophosphatase ist.

20. Verfahren nach Anspruch 16 oder 19,
dadurch gekennzeichnet,
daß das rekombinante DNA-Plasmid für die Luciferase von Vibrio harveyi oder für eine andere Bakterienluciferase oder für Käferluciferase kodiert.

21. Verfahren nach Anspruch 20,
dadurch gekennzeichnet,
daß das rekombinante DNA-Plasmid ein pCSS112-, pCSS301- oder pCSS962-Plasmid ist.

22. Verfahren nach einem der Ansprüche 19 bis 21,
dadurch gekennzeichnet,
daß Aldehyd oder Luciferin der Reaktion zugegeben werden, wenn die Menge/Aktivität der exprimierten Luciferase in den Bakterien gemessen wird.

23. Verfahren nach Anspruch 4, 5 oder 14,
dadurch gekennzeichnet,
daß die Bestimmung bei Milch, Serum oder Wasser durchgeführt wird.

## Revendications

1. Une méthode pour déterminer la présance ou la quantité d'un facteur dans un échantillon à tester, dans laquelle le facteur affecte directement ou indirectement l'ADN, l'ARN et/ou les protéines de la cellule ou les mécanismes de synthèse de celle-ci, caractérisée en ce que
a) un échantillon à tester avec une population de cellules transformées est incubé pendant une période suffisante pour permettre audit facteur, lorsqu'il est présent dans l'échantillon, d'affecter lesdites cellules, lesdites cellules étant transformées avec un plasmide ADN-recombinant, la duplication dudit plasmide étant sujette à un promoteur régulable susceptible d'être induit par un stimulus exogène indépendant de la duplication des cellules ;
b) ledit stimulus exogène est ensuite appliqué auxdites cellules pour induire la duplication dudit plasmide, à la suite de quoi le nombre de copies du plasmide commencera à augmenter dans la cellule, si la facteur n'a pas affecté le plasmide en inhibant la duplication; et
c) la modification du nombre de copies du plasmide ADN-recombinant dans la cellule est déterminée directement ou indirectement, puis comparée à un test de référence dans lequel le facteur n'était pas présent ou dans lequel il était présent en quantité connue, permettant ainsi de déterminer la présence ou la quantité dudit facteur.

2. Une méthode selon la revendication 1, caractérisée en ce que la plasmide ADN-recombinant contient une séquence ADN qui code au moins une protéine sélectionnée ou une partie de celle-ci qui est essentielle à l'activité biologique de ladite protéine, et en ce que le plasmide d'ADN-recombinant peut comporter une ou plusieurs séquences d'ADN qui rendent la cellule résistante à un antibiotique, un métal lourd ou une toxine.

3. Une méthode selon la revendication 2, caractérisée en ce que la séquence d'ADN codant la protéine est sujette à un promoteur régulable qui est commandé par rétro-activation ou par rétro-inhibition et est régulable dans lesdites cellules, et en ce qu'elle est activée simultanément ou à un moment souhaité après l'induction d'une duplication plasmidique.

4. Une méthode selon la revendication 3, caractérisée en ce que le facteur à déterminer est un facteur affectant la biosynthèse ou la duplication du plasmide ADN-recombinant, la biosynthèse de l'ARN, la transcription, la translation, les membranes cellulaires, le métabolisme ou l'activité enzymatique.

5. Une méthode selon la revendication 4, caractérisée en ce que le facteur est l'aflatoxine, un métal lourd, le bromure d'éthidium, l'acide nalidixique, le triméthoprime, la fluoroquinolone, l'aminoglucoside, la pénicilline, la céphalosporine, la rifampicine, le chloramphénicol, la tétracycline, le sulphonamide et en ce que la cellule est sensible au facteur à déterminer.

6. Une méthode selon la revendication 4 ou 5, caractérisée en ce que la cellule est Escherichia coli et le mécanisme responsable de la duplication du plasmide ADN-recombinant contenu dans les bactéries peut être régulé avec précision à l'aide d'un promoteur puissant tel que les promoteurs lambda P_{L} et P_{R}, lac, trp, et différents promoteurs hybrides tels que les promoteurs tac et des promoteurs artificiels.

7. Une méthode selon la revendication 6, caractérisée en ce que le plasmide ADN-recombinant est le pCSS123, le pCSS302 ou le pCSS305.

8. Une méthode pour déterminer la présence ou la quantité d'un facteur dans un échantillon à tester, dans laquelle le facteur affecte directement ou indirectement l'ADN, l'ARN et/ou les protéines de la cellule ou les mécanismes de synthèse de celle-ci, caractérisée en ce que :
a) un échantillon à tester avec une population de cellules transformées est incubé pendant une période suffisante pour permettre audit facteur, lorsqu'il est présent dans l'échantillon, d'affecter lesdites cellules, lesdites cellules étant transformées avec un plasmide ADN-recombinant à nombre de copies élevé contenant une séquence d'ADN qui encode une protéine de marquage ou une partie de celle-ci qui est essentielle à l'activité biologique de ladite protéine, ladite séquence étant associée à un promoteur régulable de sorte que l'expression de ladite protéine de marquage puisse être induite par un stimulus exogène ;
b) ledit stimulus exogène est ensuite appliqué à ladite population de cellules pour induire l'expression de ladite protéine de marquage, à la suite de quoi la quantité de la protéine de marquage dans la cellule commence à augmenter, si le facteur n'a pas affecté directement ou indirectement la synthèse de protéines ;
c) la quantité de la protéine de marquage exprimée par ladite population de cellules est déterminée, ladite quantité est comparée à un test de référence dans lequel le facteur n'était pas présent ou dans lequel il était présent en quantité connue, et en ce que la présence ou la quantité du facteur est donc déterminée.

9. Une méthode selon la revendication 1 ou 8, caractérisée en ce que la cellule est une bactérie gram-négative ou gram-positive telle que l'une parmi le groupe Enterobacteriaceae, de préférence le Escherichia coli, ou telle que l'une parmi le groupe Bacillus, de préférence Bacillus subtilis.

10. Une méthode selon les revendications 1 à 3, 8 ou 9, caractérisée en ce que le stimulus exogène est produit par l'ADN chromosomique de la cellule-hôte, un plasmide dans la cellule appartenant à une autre catégorie d'incompatibilité, le même plasmide, un phage lytique ou lysogénique ou un virus, ou en ce que le stimulus exogène est ajouté depuis l'extérieur des cellules, par exemple en ajoutant des composés chimiques, an modifiant la température ou par radiation.

11. Une méthode selon la revendication 10, caractérisée en ce que le plasmide ADN-recombinant peut comporter une ou plusieurs séquences d'ADN qui rendent la cellule résistante aux antibiotiques, aux métaux lourds ou aux toxines.

12. Une méthode selon la revendication 1 ou 8, caractérisée en ce que l'échantillon examiné est un aérosol sous forme gazeuse, liquide ou solide, ou l'échantillon est radioactif, ultraviolet ou une autre radiation et d'origine biologique ou non-biologique.

13. Une méthode selon la revendication 1 ou 8, caractérisée en ce que les cellules sont lyophilisées et réhydratées avant la détermination par un liquide ou milieu de culture appropriés.

14. Une méthode selon la revendication 8, caractérisée en ce que le facteur est un facteur affectant la biosynthèse de l'ADN, la biosynthèse de l'ARN, la transcription, la translation, les membranes cellulaires ou le métabolisme de la cellule contenant ledit plasmide recombinant, tel qu'un mutagène, un antibiotique, un métal lourd ou une toxine.

15. Une méthode selon la revendication 14, caractérisée en ce que la cellules est le Bacillus subtilis.

16. Une méthode selon la revendication 15, caractérisée en ce que la protéine-marqueur dans le plasmide ADN-recombinant est soumis à un promoteur puissant et régulable tel que φ 105, le promoteur de phage T5 contrôlé par l'opérateur lac, ou le promoteur régulable de saccharose, et en ce que la protéine-marqueur se trouve parmi l'alpha-amylase, la phosphatase alcaline, la β-galactosidase, la luciférase, le peroxydase, le lysozyme T4, la β-glucuronidase, l'oxydoréductase ou la pyrophosphatase.

17. Une méthode selon la revendication 14, caractérisée en ce que la cellule est le Escherichia coli.

18. Une méthode selon la revendication 6 ou 17, caractérisée en ce que le plasmide ADN-recombinant comporte une séquence d'ADN codant la protéine d'un virus, une cellule procaryotique ou une cellule eucaryotique ou sa partie essentielle par rapport à son activité biologique, et en ce que l'expression de la protéine peut être commandé à l'aide d'un promoteur régulable tel que lac, trp, P_{L} ou P_{R}, divers promoteurs hybrides tels que tac or des promoteurs artificiels.

19. Une méthode selon la revendication 18, caractérisée en ce que la protéine se trouve parmi la luciférase, la β-galactosidase, la phosphatase alcaline, le peroxydase, le lysozyme T4, la β-glucuronidase, l'oxydoréductase ou la pyrophosphatase.

20. Une méthode selon la revendication 16 ou 19, caractérisée en ce que le plasmide de ADN-recombinant code la luciférase de Vibrio harveyi ou une autre luciférase bactérienne, ou la luciférase du taupin.

21. Une méthode selon la revendication 20, caractérisée en ce que le plasmide ADN-recombinant est le plasmide pCSS112, pCSS301 ou pCSS962.

22. Une méthode selon l'une quelconque des revendications 19 à 21, caractérisée en ce que l'on ajoute de l'aldéhyde ou de la luciférine à la réaction lorsqu'on mesure la quantité/activité de la luciférase exprimée dans la bactérie.

23. Une méthode selon la revendication 4, 5 ou 14, caractérisée en ce que la détermination concerne du lait, du sérum ou de l'eau.
